# FASCICULE DE BREVET EUROPEEN

(11) **EP 1 263 721 B1**
(45) Date de publication et mention de la délivrance du brevet: **25.08.2010**
(21) Numéro de dépôt: 01909938.1
(22) Date de dépôt: 01.03.2001
(51) Int. Cl.: C07D 205/04, C07D 403/12, C07D 401/12, C07D 409/12, A61K 31/397, A61P 25/00

(54) **COMPOSITIONS PHARMACEUTIQUES CONTENANT DES DERIVES DE 3-AMINO-AZETIDINE, LES NOUVEAUX DERIVES ET LEUR PREPARATION**
3-AMINOAZETIDINDERIVATE ENTHALTENDE PHARMAZEUTISCHE ZUSAMMENSETZUNGEN, DIESE DERIVATE UND DEREN HERSTELLUNG
PHARMACEUTICAL COMPOSITIONS CONTAINING 3-AMINO-AZETIDINE DERIVATIVES, NOVEL DERIVATIVES AND PREPARATION THEREOF

(30) Priorité: 03.03.2000 FR 0002777
(43) Date de publication de la demande: 11.12.2002
(73) Titulaire: Aventis Pharma S.A., 92160 Antony (FR)
(72) Inventeur: ACHARD, Daniel, F-94320 Thiais (FR); BOUCHARD, Hervé, F-94320 Thiais (FR); BOUQUEREL, Jean, F-93700 DRANCY (FR); FILOCHE, Bruno, F-94000 Creteil (FR); GRISONI, Serge, F-94600 Choisy Le Roi (FR); HITTINGER, Augustin, F-91430 Igny (FR); MYERS, Michael, Fishers, Indiana 46038 (US)
(74) Mandataire: Rousseau, Pierrick Edouard
(86) Numéro de dépôt international: PCT/FR2001/000601
(87) Numéro de publication internationale: WO 2001/064633

(56) Documents cités:
- EP-A- 0 406 112
- WO-A-97/01556
- WO-A-99/01451
- US-A- 4 242 261

## Description

La présente invention concerne des compositions pharmaceutiques contenant comme ingrédient actif un composé de formule : 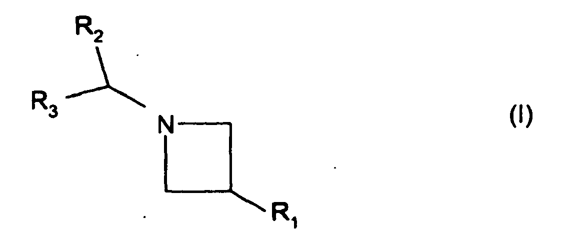 ou un de ses sels pharmaceutiquement acceptables, les nouveaux dérivés de formule (I), leurs sels pharmaceutiquement acceptables et leur préparation.

Le composé de formule (I) pour lequel R₂ et R₃ représentent des radicaux phényle, R₁ représente un radical -N(R₅)-Y-R₆, Y est SO₂, R₅ représente un radical méthyle et R₆ représente un radical phényle est décrit comme intermédiaire de synthèse dans le brevet W099101451. Les autres composés et leurs sels pharmaceutiquement acceptables sont nouveaux et en tant que tels font partie de l'invention.

Dans la formule (I)
R₁ représente un radical -NHCOR₄ ou -N(R₅)-Y-R₆,
Y est CO ou SO₂,
R₂ et R₃, identiques ou différents, représentent soit un aromatique choisi parmi phényle, naphtyle et indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, -COOalk, -CONR₇R₈, -CO-NH-NR₉R₁₀, alkylsulfanyle, alkylsulfinyle, alkylsulfonyle, alkylsulfanylalkyle, alkylsulfinylalkyle, alkylsulfonylalkyle, hydroxyalkyle, ou -alk-NR₇R₈; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, pyrimidinyle, furyle, imidazolyle, isochromannyle, isoquinolyle, pyrrolyle, pyridyle, quinolyle, 1,2,3,4-tétrahydroisoquinolyle, thiazolyle et thiényle, ces hétéroaromatiques pouvant être non substitués ou substitués par un halogène, alkyle, alcoxy, hydroxy, trifluorométhyle, trifluorométhoxy, cyano, -COOH, -COOalk, -CO-NH-NR₉R₁₀, -CONR₇R₈, -alk-NR₉R₁₀, alkylsulfanyle, alkylsulfinyle, alkylsulfonyle, alkylsulfanylalkyle, alkylsulfinylalkyle, alkylsulfonylalkyle ou hydroxyalkyle,
R₄ représente un radical -alk-SO₂-R₁₁, -alk-SO₂-CH=CH-R₁₁, Het substitué par -SO₂ R₁₁ ou phényle substitué par -SO₂-R₁₁ ou -alk-SO₂-R₁₁,
R₅ représente un atome d'hydrogène ou un radical alkyle,
R₆ représente un radical phénylalkyle, Het ou Ar,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents représentent un atome d'hydrogène ou un radical alkyle, -COOalk, cycloalkyle, alkylcycloalkyle, -alk-O-alk ou hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, -CS-NHalk, oxo, hydroxyalkyle, -alk-O-alk ou -CO-NH₂,
R₁₁ représente un radical alkyle, Ar ou Het,
Ar représente un radical phényle, naphtyle ou indènyle, ces radicaux étant éventuellement substitués par un ou plusieurs halogène, alkyle, alcoxy, cyano, -CO-alk, -COOH, -COOalk, -CONR₁₂R₁₃, -CO-NH-NR₁₄R₁₅, alkylsulfanyle, alkylsulfinyle, alkylsulfonyle, -alk-NR₁₄R₁₅, -NR₁₄R₁₅, alkylthioalkyle, formyle, hydroxy, hydroxyalkyle, Het, -O-alk-NH-cycloalkyle, OCF₃, CF₃, -NH-CO-alk, -SO₂NH₂, -NH-COCH₃, -NH-COOalk, Het ou bien sur 2 atomes de carbone adjacents par un dioxyméthylène,
Het représente un hétérocycle mono ou bicyclique insaturé ou saturé, ayant 3 à 10 chaînons et contenant un ou plusieurs hétéroatomes choisis parmi oxygène, soufre et azote éventuellement substitué par un ou plusieurs alkyle, alcoxy, vinyle, halogène, alcoxycarbonyle, oxo, hydroxy, OCF₃ ou CF₃, les hétérocycles azotés étant éventuellement sous leur forme N-oxydée,
R₁₂ et R₁₃, identiques ou différents, représentent un atome d'hydrogène ou un radical alkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₁₄ et R₁₅, identiques ou différents, représentent un atome d'hydrogène ou un radical alkyle, -COOalk, cycloalkyle, alkylcycloalkyle, -alk-O-alk, hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, hydroxyalkyle, -alk-O-alk, -CO-NH₂,
alk représente un radical alkyle ou alkylène.

Dans les définitions précédentes et celles qui suivent, sauf mention contraire, les radicaux et portions alkyle et alkylène et les radicaux et portions alcoxy sont en chaîne droite ou ramifiée et contiennent 1 à 6 atomes de carbone et les radicaux cycloalkyle contiennent 3 à 10 atomes de carbone.

Parmi les radicaux alkyle on peut citer les radicaux méthyle, éthyle, n-propyle, isopropyle, n-butyle, sec-butyle, iso-butyle, tert-butyle, pentyle, hexyle. Parmi les radicaux alcoxy on peut citer les radicaux méthoxy, éthoxy, n-propoxy, iso-propoxy, n-butoxy, iso-butoxy, sec-butoxy, tert-butoxy, pentyloxy.

Parmi les radicaux cycloalkyle, on peut citer les radicaux cyclopropyle, cyclobutyle, cyclopentyle, cyclohexyle.

Le terme halogène comprend chlore, fluor, brome et iode.

Parmi les hétérocycles réprésentés par Het, on peut citer les hétérocycles suivants : benzimidazole, benzoxazole, benzothiazole, benzothiophène, cinnoline, thiophène, quinazoline, quinoxaline, quinoline, pyrazole, pyrrole, pyridine, imidazole, indole, isoquinoline, pyrimidine, thiazole, thiadiazole, pipéridine, pipérazine, pyrrolidine, triazole, furane, tétrahydroisoquinoline, tétrahydroquinoline, ces hétérocycles étant éventuellement substitués par un ou plusieurs alkyle, alcoxy, vinyle, halogène, alcoxycarbonyle, oxo, hydroxy, OCF₃ ou CF₃.

Les composés de formule (I) peuvent se présenter sous forme d'énantiomères et de diastéréoisomères. Ces isomères optiques et leurs mélanges font également partie de l'invention.

De façon préférentielle, les composés de formule (I) sont ceux pour lesquels
R, représente un radical -N(R₉)-Y-R₆,
Y est SO₂,
R₂ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidinyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyle ou hydroxyalkyle,
R₃ représente soit un phényle non substitué ou substitué par un ou plusieurs halogène, alkyle, alcoxy, trifluorométhyle, trifluorométhoxy, cyano, -CONR₇R₈, hydroxyalkyle ou -a'k-NR₇R₈; soit un hétéroaromatique choisi parmi les cycles pyridyle, pyrimidinyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyleou hydroxyalkyle,
R₅ représente un atome d'hydrogène ou alkyle,
R₆ représente un radical naphtyle, phénylalkyle, Het ou phényle éventuellement substitué par un ou plusieurs halogène, alkyle, alcoxy, cyano, -CO-alk, COOalk, -CONR₁₂R₁₃, -alk-NR₁₄R₁₅, -NR₁₄R₁₅, hydroxy, hydroxyalkyle, Het, OCF₃. CF₃, -NH-CO-alk, -SO₂NH₂, -NH-COOalk, ou bien sur 2 atomes de carbone adjacents par dioxyméthylène,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents représentent un atome d'hydrogène ou un radical alkyle, cycloalkyle, alkylcycloalkyle ou hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, oxo ou -CO-NH₂,
R₁₂ et R₁₃, identiques ou différents, représentent un atome d'hydrogène ou un radical alkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₁₄ et R₁₅ identiques ou différents, représentent un atome d'hydrogène ou un radical alkyle, cycloalkyle, alkylcycloalkyle ou hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, oxo, hydroxyalkyle ou -CO-NH₂,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, vinyle, halogène, alcoxycarbonyle, oxo, hydroxy, les hétérocycles azotés étant éventuellement sous leur forme N-oxydée et, de préférence, Het représente un hétérocycle choisi parmi les hétérocycles suivants : benzimidazole, benzoxazole, benzothiazole, benzothiophène, thiophène, quinazoline, quinoxaline, quinoline, pyrrole, pyridine, imidazole, indole, isoquinoline, pyrimidine, thiazole, thiadiazole, furane, tétrahydroisoquinoline et tétrahydroquinoline, ces hétérocycles étant éventuellement substitués par un ou plusieurs alkyle, alcoxy, vinyle, halogène, oxo, hydroxy, OCF₃ ou CF₃.

Encore plus préférentiellement, les composés de formule (I) sont choisis parmi les composés suivants :
R₁ représente un radical -N(R₅)-Y-R₆,
Y est SO₂,
R₂ représente soit un phényle non substitué ou substitué par un ou plusieurs halogène, alkyle, alcoxy, trifluorométhyle, trifluorométhoxy ou hydroxyalkyle; soit un hétéroaromatique choisi parmi les cycles pyridyle et pyrimidyle, ces hétéroaromatiques pouvant être non substitués ou substitués par un halogène, alkyle, alcoxy, hydroxy, trifluorométhyle ou trifluorométhoxy,
R₃ représente soit un phényle non substitué ou substitué par un ou plusieurs halogène, alkyle, alcoxy, trifluorométhyle, trifluorométhoxy, hydroxyalkyle; soit un hétéroaromatique choisi parmi les cycles pyridyle et pyrimidyle, ces hétéroaromatiques pouvant être non substitués ou substitués par un halogène, alkyle, alcoxy, hydroxy, trifluorométhyle ou trifluorométhoxy,
R₅ représente un atome d'hydrogène ou alkyle,
R₆ représente un radical naphtyle, phénylalkyle, Het ou phényle éventuellement substitué par un ou plusieurs halogène, alkyle, alcoxy, -NR₁₄R₁₅, hydroxy, hydroxyalkyle, OCF₃, CF₃ ou -SO₂NH₂, ou bien sur 2 atomes de carbone adjacents par dioxyméthylène R₁₄ et R₁₅, identiques ou différents représentent un atome d'hydrogène ou un radical alkyle, cycloalkyle, alkylcycloalkyle ou hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, oxo, hydroxyalkyle ou -CO-NH₂,

Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, vinyle, halogène, alcoxycarbonyle, oxo, hydroxy, les hétérocycles azotés étant éventuellement sous leur forme N-oxydée et, de préférence, Het représente un hétérocycle choisi parmi les hétérocycles suivants : benzimidazole, benzoxazole, benzothiazole, benzothiophène, thiophène, quinoline, pyrrole, pyridine, pyrimidine, thiazole, thiadiazole, furane, tétrahydroisoquinoline, tétrahydroquinoline, ces hétérocycles étant éventuellement substitués par un ou plusieurs alkyle, alcoxy, vinyle, halogène, oxo, hydroxy, OCF₃ ou CF₃.

Les composés de formule (I) pour lesquels R₁ représente un radical -NHCOR₄ peuvent être préparés selon le schéma réactionnel suivant :

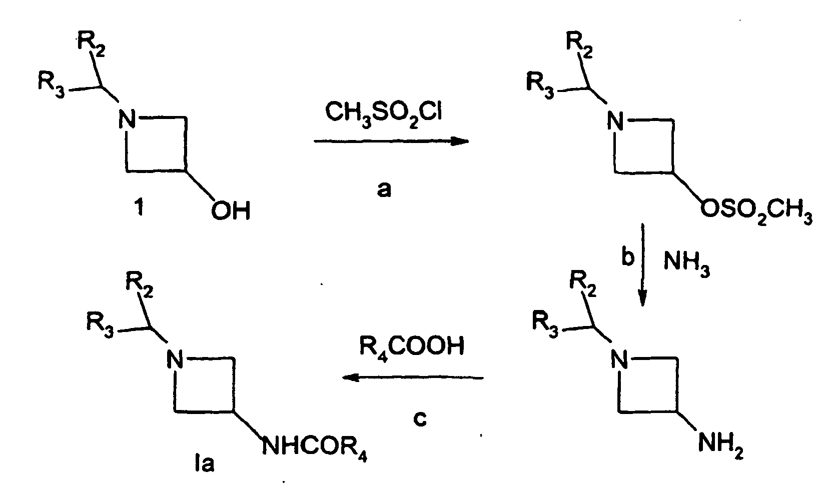

Dans ces formules R₂, R₃ et R₄ ont les mêmes significations que dans la formule (I).

L'étape a s'effectue généralement au sein d'un solvant inerte tel que le tétrahydrofuranne, le dioxanne, un solvant chloré (dichlorométhane, chloroforme par exemple), à une température comprise entre 15°C et 30°C, en présence d'une base telle qu'une trialkyamine (triéthylamine, dipropyléthylamine par exemple) ou au sein de la pyridine, à une température comprise entre 0°C et 30°C,.

L'étape b s'effectue de préférence, au sein du méthanol, en autoclave, à une température comprise entre 50 et 70°C.

L'étape c s'effectue généralement en présence d'un agent de condensation utilisé en chimie peptidique tel qu'un carbodiimide (par exemple le 1-(3-diméthylaminopropyl)-3-éthylcarbodiimide, le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température d'ébullition du mélange réactionnel. On peut également utiliser un dérivé réactif de l'acide comme un chlorure d'acide, éventuellement en présence d'un accepteur d'acide tel qu'une base organique azotée (trialkylamine, pyridine, diaza-1,8 bicyclo[5.4.0]undécène-7 ou diaza-1,5 bicyclo[4.3.0]nonène-5 par exemple), dans un solvant tel que cité ci-dessus, ou un mélange de ces solvants, à une température comprise entre 0°C et la température d'ébullition du mélange réactionnel.

Les dérivés R₄COOH sont commerciaux ou peuvent être obtenus selon les méthodes décrites dans R.C. LAROCK, Comprehensive Organic Transformations, VCH editor.

Les azétidinols de formule 1 peuvent être obtenus par application ou adaptation des méthodes décrites par KATRITZKY A.R et coll., J. Heterocycl. Chem., 271 (1994), ou DAVE P.R., J. Org. Chem., 61, 5453 (1996) et dans les exemples. On opère généralement selon le schéma réactionnel suivant : 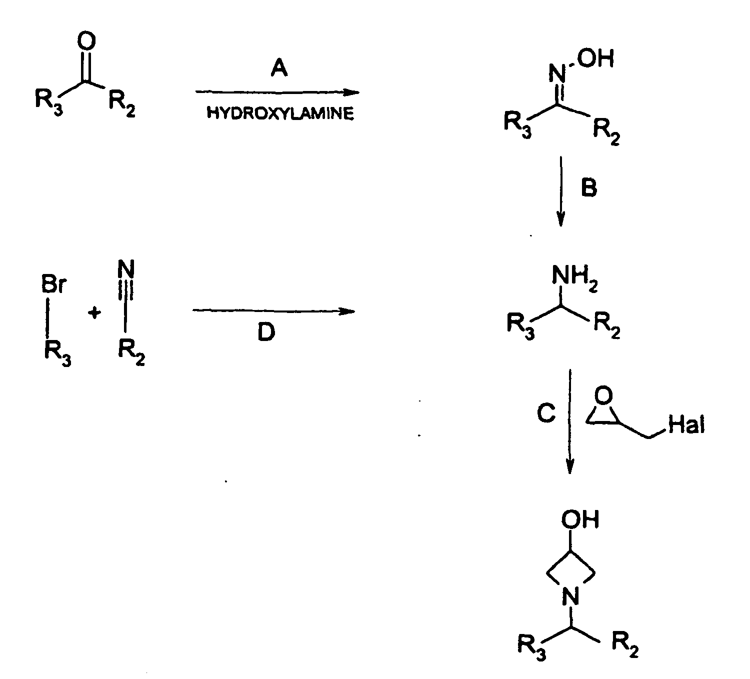 dans ces formules R₂ et R₃ ont les mêmes significations que dans la formule (I) et Hal représente un atome de chlore ou de brome.

Dans l'étape A, on opère de préférence au sein d'un solvant inerte tel qu'un alcool aliphatique 1-4C (éthanol, méthanol par exemple), éventuellement en présence d'un hydroxyde de métal alcalin, à la température d'ébullition du milieu réactionnel.

Dans l'étape B, la réduction s'effectue généralement, au moyen d'hydrure de lithium et d'aluminium, au sein du tétrahydrofuranne à la température d'ébullition du milieu réactionnel.

Dans l'étape C, on opère de préférence au sein d'un solvant inerte tel qu'un alcool aliphatique 1-4C (éthanol, méthanol par exemple), en présence d'hydrogénocarbonate de sodium, à une température comprise entre 20°C et la température d'ébullition du milieu réactionnel.

Dans l'étape D, on opère selon la méthode décrite par GRISAR M. et coll. dans J. Med. Chem., 885 (1973). On forme le magnésien du dérivé bromé puis on fait réagir le nitrile, au sein d'un éther tel que l'éther éthylique, à une température comprise entre 0°C et la température d'ébullition du milieu réactionnel. Après hydrolyse avec un alcool, l'imine intermédiaire est réduite *in situ* par du borohydrure de sodium à une température comprise entre 0°C et la température d'ébullition du milieu réactionnel.

Les dérivés R₂-CO-R₃ sont commercialisés ou peuvent être obtenus par application ou adaptation des méthodes décrites par KUNDER N.G. et coll. J. Chem. Soc. Perkin Trans 1, 2815 (1997); MORENO-MARRAS M., Eur. J. Med. Chem., 23 (5) 477 (1988); SKINNER et coll., J. Med. Chem., 14 (6) 546 (1971); HURN N.K., Tet. Lett., 36 (52) 9453 (1995); MEDICI A. et coll., Tet. Lett., 24 (28) 2901 (1983); RIECKE R.D. et coll., J. Org. Chem., 62 (20) 6921 (1997); KNABE J. et coll., Arch. Pharm., 306 (9) 648 (1973); CONSONNI R. et coll., J. Chem. Soc. Perkin Trans 1, 1809 (1996); FR-96-2481 et JP-94-261393.

Les dérivés R₃Br sont commercialisés ou peuvent être obtenus par application ou adaptation des méthodes décrites par BRANDSMA L. et coll., Synth. Comm., 20 (11) 1697 et 3153 (1990); LEMAIRE M. et coll., Synth. Comm., 24 (1) 95 (1994); GODA H. et coll., Synthesis, 9 849 (1992); BAEUERLE P. et coll., J. Chem. Soc. Perkin Trans 2, 489 (1993).

Les dérivés R₂CN sont commercialisés ou peuvent être obtenus par application ou adaptation des méthodes décrites par BOUYSSOU P. et coll., J. Het. Chem., 29 (4) 895 (1992); SUZUKI N. et coll., J. Chem. Soc. Chem. Comm., 1523 (1984); MARBURG S. et coll., J. Het. Chem., 17 1333 (1980); PERCEC V. et coll., J. Org. Chem., 60 (21) 6895 (1995).

Les composés de formule (I) pour lesquels R₁ représente un radical -N(R₅)-Y-R₆ peuvent être préparés selon le schéma réactionnel suivant : 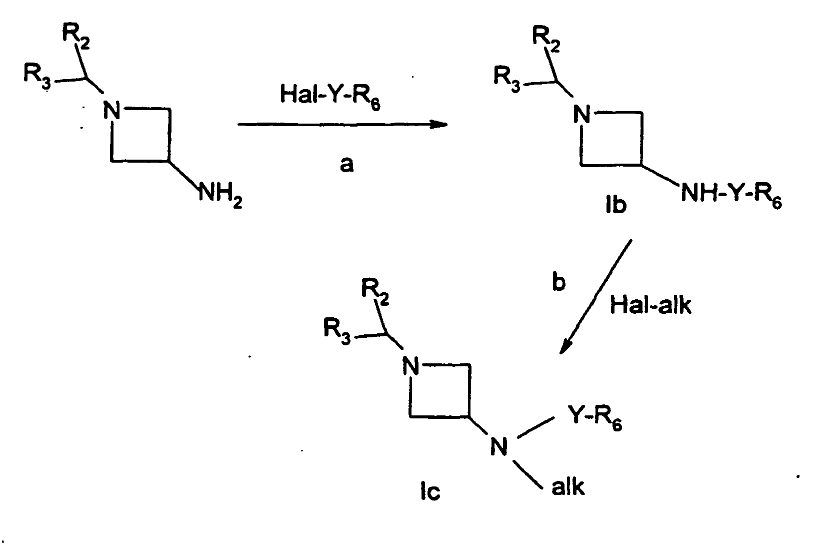 dans ces formules Y, R₂, R₃ et R₆ ont les mêmes significations que dans la formule (I), Hal représente un atome d'halogène et, de préférence, un atome d'iode, de chlore ou de brome;

L'étape a s'effectue généralement au sein d'un solvant inerte tel que le tétrahydrofuranne, le dioxanne, un solvant chloré (dichlorométhane, chloroforme par exemple), en présence d'une amine telle qu'une trialkylamine (triéthylamine par exemple), à une température comprise entre 5°C et 20°C.

L'étape b s'effectue généralement au sein d'un solvant inerte tel que le tétrahydrofuranne, en présence d'hydrure de sodium, à une température 0°C et la température d'ébullition du milieu réactionnel.

Les dérivés Hal-SO₂R₆ sont commercialisés ou peuvent être obtenus par halogénation des acides sulfoniques correspondants, notamment in situ en présence de chlorosulfonylisocyanate et d'alcool, au sein d'un solvant halogéné (dichlorométhane, chloroforme par exemple).

Les dérivés Hal-CO-R₆ sont commerciaux ou peuvent être préparés selon les méthodes décrites dans R.C. LAROCK, Comprehensive Organic Transformations, VCH editor.

Les composés de formule (I) peuvent également être préparés selon le schéma réactionnel suivant :

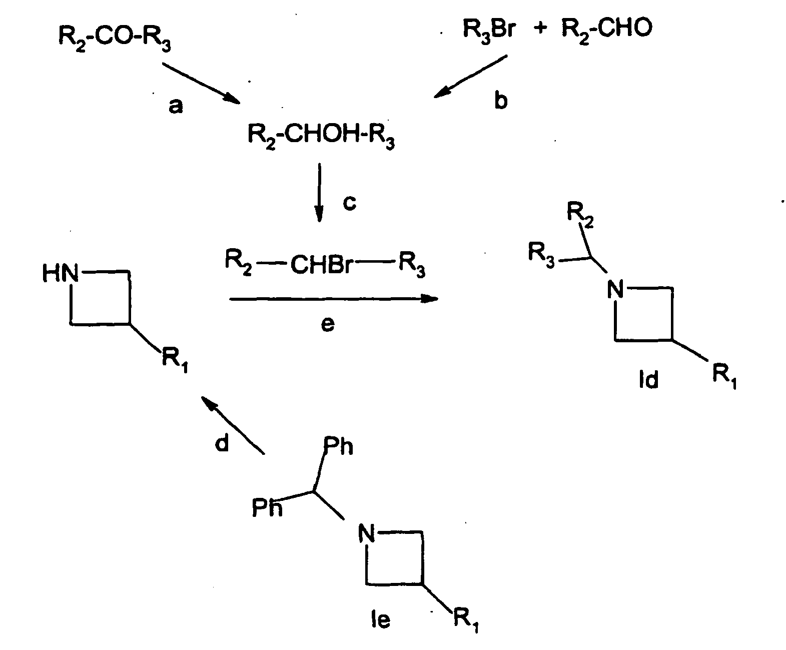

Dans ces formules R₁, R₂ et R₃ ont les mêmes significations que dans la formule (I) et Ph représente un phényle.

L'étape a s'effectue généralement au sein d'un alcool tel que le méthanol, en présence de borohydrure de sodium, à une température voisine de 20°C. Dans l'étape b, on prépare le magnésien du dérivé bromé et le fait réagir, au sein d'un solvant inerte tel que l'éther éthylique ou le tétrahydrofuranne, à une température comprise entre 0°C et la température d'ébullition du milieu réactionnel.

L'étape c s'effectue au moyen d'un agent d'halogénation tel que l'acide bromhydrique, le bromure de thionyle, le chlorure de thionyle, un mélange de triphénylphosphine et de tétrabromure ou tétrachlorure de carbone, au sein de l'acide acétique ou un solvant inerte tel que le dichlorométhane, le chloroforme, le tétrachlorure de carbone ou le toluène, à une température comprise entre 0°C et la température d'ébullition du milieu réactionnel.

L'étape d s'effectue au moyen d'hydrogène, en présence de charbon palladié, au sein d'un alcool tel que le méthanol, à une température voisine de 20°C.

L'étape e s'effectue au sein d'un solvant inerte tel que l'acétonitrile, en présence d'un carbonate de métal alcalin (carbonate de potassium par exemple), et d'iodure de potassium, à une température comprise entre 20°C et la température d'ébullition du milieu réactionnel.

Les dérivés R₃Br et les dérivés R₂-CHO sont commercialisés ou peuvent être obtenus selon les méthodes décrites par exemple par R.C. LAROCK, Comprehensive Organic Transformations, VCH editor.

Les composés de formule (I) pour lesquels R, représente un radical -N(R₅)-Y-R₆ dans lequel R₆ est un radical phényle substitué par hydroxy peuvent également être préparés par hydrolyse d'un composé de formule (I) correspondant pour lequel R₁ représente un radical -N(R₅)-Y-R₆ dans lequel R₆ est un radical phényle substitué par alcoxy.

Cette hydrolyse s'effectue généralement au sein d'un solvant inerte tel qu'un solvant chloré (dichlorométhane, chloroforme par exemple), au moyen de tribromure de bore, à une température voisine de 20°C.

Les composés de formule (I) pour lesquels R, représente un radical -N(R₅)-Y-R₆ dans lequel R₆ est un radical phényle substitué par hydroxyalkyle(1C) peuvent également être préparés par action de l'hydrure de diisobutylaluminium sur un composé de formule (I) correspondant pour lequel R₁ représente un radical -N(R₅)-Y-R₆ dans lequel R₆ est un radical phényle substitué par alcoxycarbonyle.

Cette réaction s'effectue généralement au sein d'un solvant inerte tel que le toluène, au moyen d'hydrure de diisopropylaluminium, à une température comprise entre -50°C et 25°C.

Les composés de formule (I) pour lesquels R₁ représente un radical -N(R₅)-Y-R₆ dans lequel R₆ est un radical phényle substitué par pyrrolidinyl-1 peuvent également être préparés par action de pyrrolidine et d'un composé de formule (I) correspondant pour lequel R₁ représente un radical -N(R₅)-Y-R₆ dans lequel R₆ est un radical phényle substitué par fluor.

Cette réaction s'effectue de préférence au sein d'un solvant inerte tel que le diméthylsulfoxyde, à une température de 90°C.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane ou d'allyle au moyen de catalyseurs du palladium. Comme exemples de groupes protecteurs de la fonction hydroxy, on peut citer les triéthylsilyle, tert-butyldiméthylsilyle qui peuvent être régénérés au moyen de fluorure de tétrabutylammonium ou bien les acétals dissymétriques (méthoxyméthyle, tétrahydropyranyle par exemple) avec régénération au moyen d'acide chlorhydrique. Comme groupes protecteurs des fonctions carboxy, on peut citer les esters (allyle, benzyle par exemple), les oxazoles et les 2-alkyl-1,3-oxazolines. D'autres groupes protecteurs utilisables sont décrits par GREENE T.W. et coll., Protecting Groups in Organic Synthesis, second edition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) présentent des propriétés pharmacologiques intéressantes. Ces composés possèdent une forte affinité pour les récepteurs cannabinoïdes et particulièrement ceux de type CB1. Ce sont des antagonistes du récepteur CB1 et sont donc utiles dans le traitement et la prévention des désordres touchant au système nerveux central, au système immunitaire, au système cardio-vasculaire ou endocrinien, au système respiratoire, à l'appareil gastrointestinal et aux désordres de la reproduction (Hollister, Pharm. Rev.; 38, 1986, 1-20, Reny et Sinha, Prog. Drug Res., 36, 71-114 (1991), Consroe et Sandyk, in Marijuana/Cannabinoids, Neurobiology and Neurophysiology, 459, Murphy L. and Barthe A. Eds, CRC Press, 1992).

C'est ainsi que ces composés peuvent être utilisés pour le traitement ou la prévention des psychoses y compris la schizophrénie, des troubles anxieux, de la dépression, de l'épilepsie, de la neurodégénération, des désordres cérébelleux et spinocérébelleux, des désordres cognitifs, du trauma crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques. Ils peuvent également être utilisés pour le traitement ou la prévention du transit intestinal.

L'affinité des composés de formule (I) pour les récepteurs du cannabis a été déterminée selon la méthode décrite par KUSTER J.E., STEVENSON J.I., WARD S.J., D'AMBRA T.E., HAYCOCK D.A. dans J. Pharmacol. Exp. Ther., 264 1352-1363 (1993).

Dans ce test, la Cl₅₀ des composés de formule (I) est inférieure ou égale à 1000 nM.

Leur activité antagonistique a été montrée au moyen du modèle d'hypothermie induite par un agoniste des récepteurs du cannabis (CP-55940) chez la souris, selon la méthode décrite par Pertwee R.G. dans Marijuana, Harvey D.J. eds, 84 Oxford IRL Press, 263-277 (1985).

Dans ce test, la DE50 des composés de formule (I) est inférieure ou égale à 50 mg/kg.

Les composés de formule (I) présentent une toxicité faible. Leur DL₅₀ est supérieure à 40 mg/kg par voie sous cutanée chez la souris.

Les exemples suivants illustrent l'invention.

### Exemple 1

A une solution de 61,4 mg de 1-[bis(4-chlorophényl)méthyl]azétidin-3-yl-amine dans 3 cm³ de dichlorométhane on ajoute, à température ambiante et sous atmosphère d'argon, successivement 69,3 mm³ de triéthylamine et 110 mg de chlorure de thièn-2-yl-sulfonyle. Après 68 heures d'agitation à température ambiante, le mélange réactionnel est introduit sur une cartouche Bond Elut^{®} SCX (3 cm³/500 mg), en éluant successivement par deux fois 2 cm³ de dichlorométhane, puis deux fois 2 cm³ de solution 1 M d'ammoniac dans le méthanol. Les fractions ammoniacales sont jointes et concentrées à sec sous pression réduite (2,7 kPa). Le résidu obtenu est dissous dans 5 cm³ de dichlorométhane, lavé avec trois fois 3 cm³ d'eau distillée, séché sur sulfate de magnésium, filtré et concentré à sec sous pression réduite (2,7 kPa). On obtient ainsi 60 mg de N-{1-[bis(4-chlorophényl)méthyl]azétidin-3-yl}-thièn-2-yl-sulfonamide sous forme d'une meringue de couleur crème [Spectre de R.M.N. ¹H (300 MHz, CDCl₃, δ en ppm) : 2,77 (t dédoublé, J = 7 et 2 Hz : 2H); 3,40 (t dédoublé, J = 7 et 2 Hz : 2H); 4,06 (mt : 1H); 4,21 (s : 1H); de 4,85 à 5,25 (mf étalé : 1H); 7,06 (t, J = 4,5 Hz : 1H); de 7,15 à 7,35 (mt : 8H); 7,58 (mt : 2H)].

La 1-[bis(4-chlorophényl)méthyl]azétidin-3-yl-amine peut être obtenue de la manière suivante : A 27 g de méthylsulfonate de 1-[bis(4-chlorophényl)méthyl]azétidin-3-yle contenus dans un autoclave préalablement refroidi vers -60°C on ajoute 400 cm³ d'un mélange de méthanol et d'ammoniac liquide (50/50 en volumes). Le milieu réactionnel est ensuite agité à 60°C pendant 24 heures, puis abandonné à l'air libre pour permettre l'évaporation de l'ammoniac et enfin concentré sous pression réduite (2,7 kPa). Le résidu est repris par 500 cm³ d'une solution aqueuse 0,37N d'hydroxyde de sodium et extrait par quatre fois 500 cm³ d'éther éthylique. Les phases organiques réunies sont lavées successivement avec deux fois 100 cm³ d'eau distillée et 100 cm³ d'une solution saturée de chlorure de sodium, séchées sur du sulfate de magnésium, filtrées et concentrées sous pression réduite (2,7 kPa). Le résidu obtenu est purifié par chromatographie-flash sur gel de silice [étant: dichlorométhane/méthanol (95/5 en volumes)]. On obtient 14,2 g de 1-[bis(4-chlorophényl)méthyl]azétidin-3-yl-amine sous forme d'une huile, qui concrétise en un solide de couleur crème.

Le méthylsulfonate de 1-[bis(4-chlorophényl)méthyl]azétidin-3-yle peut être préparé de la façon suivante : A une solution de 12 g de 1-[bis-(4-chlorophényl)méthyl]azétidin-3-ol dans 200 cm³ de dichlorométhane, on ajoute sous argon en 10 minutes 3,5 cm³ de chlorure de méthylsulfonyle, puis refroidit à +5°C et coule en 10 minutes 3,8 cm³ de pyridine. Après 30 minutes d'agitation à +5°C puis 20 heures à 20°C, le mélange réactionnel est dilué avec 100 cm³ d'eau et 100 cm³ de dichlorométhane. Le mélange, d'abord filtré est décanté. La phase organique est lavée avec de l'eau, puis séchée sur sulfate de magnésium, filtrée, et concentrée à sec sous pression réduite (2,7 kPa). L'huile obtenue est chromatographiée sur une colonne de gel de silice (granulométrie 0,063-0,200 mm, hauteur 40 cm, diamètre 3,0 cm), en éluant sous une pression de 0,5 bar d'argon avec un mélange de cyclohexane et d'acétate d'éthyle (70/30 en volumes) et en recueillant des fractions de 100 cm³. Les fractions 4 à 15 sont réunies et concentrées à sec sous pression réduite (2,7 kPa). On obtient 6,8 g d'ester 1-[bis-(4-chlorophényl)méthyl]azétidin-3-yl de l'acide méthylsulfonique, sous la forme d'une huile jaune.

Le 1-[bis(4-chlorophényl)méthyl]azétidin-3-ol peut être préparé selon le mode opératoire décrit par KATRITZKY A.R. et coll., J. Heterocycl. Chem., 271 (1994), en partant de 35,5 g de chlorhydrate de [bis(4-chlorophényl)méthyl]amine et 11,0 cm³ d'épichlorhydrine. On isole 9,0 g de 1-[bis(4-chlorophényl)méthyl]azéfidin-3-ol.

Le chlorhydrate de [bis(4-chlorophényl)méthyl]amine peut être préparé selon la méthode décrite par GRISAR M. et coll., J. Med. Chem., 885 (1973).

### Exemple 2

En opérant selon le mode opératoire de l'exemple 1 mais à partir de 124 mg de chlorure de 4-méthoxyphénylsulfonyle, on obtient 12 mg de N-{1-[bis(4-chlorophényl)méthyl]azétidin-3-yl}-4-méthoxyphénylsulfonamide sous forme d'une laque de couleur crème [Spectre de R.M.N. ¹H (300 MHz, CDCl₃, δ en ppm) : 2,70 (t dédoublé, J = 7 et 2 Hz : 2H); 3,35 (t dédoublé, J = 7 et 2 Hz : 2H); 3,85 (s : 3H); 3,94 (mt : 1H); 4,18 (s : 1H); 4,83 (d, J = 9 Hz : 1H); 6,94 (d large, J = 9 Hz : 2H); 7,22 (s : 8H); 7,75 (d large, J = 9 Hz : 2H)].

### Exemple 3

En opérant selon le mode opératoire de l'exemple 1 mais à partir de 140 mg de chlorure de 4-acétamidophénylsulfonyle, on obtient 13 mg de N-[4-(N-{1-[bis(4-chlorophényl)méthyl]azétidin-3-yl}sulfamoyl)phényl]acétamide sous forme d'une laque de couleur crème [Spectre de R.M.N. ¹H (300 MHz, CDCl₃, δ en ppm) : 2,26 (s : 3H); 2,74 (t dédoublé, J = 7 et 2 Hz : 2H); 3,39 (t dédoublé, J = 7 et 2 Hz : 2H); 4,01 (mt : 1H); 4,22 (s : 1H); 4,92 (d, J = 9 Hz : 1H); 7,32 (mt : 8H); 7,49 (s large : 1H); 7,68 (d large, J = 9 Hz : 2H); 7,81 (d large, J = 9 Hz : 2H)].

### Exemple 4

En opérant selon le mode opératoire de l'exemple 1 mais à partir de 114 mg de chlorure de 4-méthylphénylsulfonyle, on obtient 19 mg de N-{1-[bis(4-chlorophényl)méthyl]azétidin-3-yl}-4-méthylphénylsulfonamide sous forme d'une laque incolore [Spectre de R.M.N. ¹H (300 MHz, CDCl₃, δ en ppm) : 2,42 (s : 3H); 2,71 (t dédoublé, J = 7 et 2 Hz : 2H); 3,36 (t dédoublé, J = 7 et 2 Hz : 2H); 3,97 (mt : 1H); 4,19 (s : 1H); 4,81 (d, J = 9,5 Hz : 1H); de 7,15 à 7,40 (mt : 10H); 7,71 (d large, J = 8,5 Hz : 2H)].

### Exemple 5

En opérant selon le mode opératoire de l'exemple 1 mais à partir de 142 mg de chlorure de 3,4-diméthoxyphénylsulfonyle, on obtient 10 mg de N-{1-[bis(4-chlorophényl)méthyl]azétidin-3-yl}-3,4-diméthoxyphénylsulfonamide sous forme d'une laque de couleur crème [Spectre de R.M.N. ¹H (300 MHz, CDCl₃, δ en ppm) : 2,72 (t large, J = 7,5 Hz : 2H); 3,37 (t large, J = 7,5 Hz : 2H); de 3,85 à 4,00 (mt : 1H); 3,91 (s : 3H); 3,93 (s : 3H); 4,19 (s : 1H); 4,84 (d, J = 9 Hz : 1H); 6,90 (d, J = 8,5 Hz : 1H); 7,23 (mt : 8H); 7,29 (d, J = 2 Hz : 1H); 7,43 (dd, J = 8,5 et 2 Hz : 1H)].

### Exemple 6

En opérant selon le mode opératoire de l'exemple 1 mais à partir de 117 mg de chlorure de 3-fluorophénylsulfonyle, on obtient 13,5 mg de N-{1-[bis(4-chlorophényl)méthyl]azétidin-3-yl}-3-fluorophénylsulfonamide sous forme d'une laque de couleur crème [Spectre de R.M.N. ¹H (400 MHz, CDCl₃, δ en ppm) : 2,79 (t dédoublé, J = 7 et 2 Hz : 2H); 3,43 (t dédoublé, J = 7 et 2 Hz : 2H); 4,05 (mf : 1H); 4,24 (s : 1H); 4,91 (mf : 1H); de 7,20 à 7,40 (mt : 9H); de 7,50 à 7,65 (mt : 2H); 7,67 (d large, J = 8 Hz : 1H)].

### Exemple 7

En opérant selon le mode opératoire de l'exemple 1 mais à partir de 147 mg de chlorure de 3,4-dichlorophénylsulfonyle, on obtient 20 mg de N-{1-[bis(4-chlorophényl)méthyl]azétidin-3-yl}-3,4-dichlorophénylsulfonamide sous forme d'une laque de couleur crème [Spectre de R.M.N. ¹H (300 MHz, CDCl₃, δ en ppm) : 2,77 (t dédoublé, J = 7 et 2 Hz : 2H); 3,40 (t dédoublé, J = 7 et 2 Hz : 2H); 3,98 (mt : 1H); 4,21 (s : 1H); de 4,85 à 5,15 (mf : 1H); de 7,20 à 7,35 (mt : 8H); 7,57 (d, J = 8,5 Hz : 1H); 7,65 (dd, J = 8,5 et 2 Hz : 1H); 7,93 (d, J = 2 Hz : 1H)].

### Exemple 8

En opérant selon le mode opératoire de l'exemple 1 mais à partir de 121 mg de chlorure de 3-cyanophénylsulfonyle, on obtient 21 mg de N-{1-[bis(4-chlorophényl)méthyl]azétidin-3-yl}-3-cyanophénylsulfonamide sous forme d'une laque de couleur crème [Spectre de R.M.N. ¹H (300 MHz, CDCl₃, δ en ppm) : 2,76 (t dédoublé, J = 7 et 2 Hz : 2H); 3,39 (t dédoublé, J = 7 et 2 Hz : 2H); 3,99 (mt : 1H); 4,21 (s : 1H); de 4,80 à 5,60 (mf très étalé : 1H); de 7,15 à 7,35 (mt : 8H); 7,65 (t, J = 8 Hz : 1H); 7,86 (d large, J = 8 Hz : 1H): 8,05 (d large, J = 8 Hz : 1H); 8,13 (s large : 1H)].

### Exemple 9

En opérant selon le mode opératoire de l'exemple 1 mais à partir de 142 mg de chlorure de 2,5-diméthoxyphénylsulfonyle, on obtient 31 mg de N-{1-[bis(4-chlorophényl)méthyl]azétidin-3-yl}-2,5-diméthoxyphénylsulfonamide sous forme d'une laque de couleur crème [Spectre de R.M.N. ¹H (300 MHz, CDCl₃, δ en ppm) : 2,73 (t dédoublé, J = 7 et 2 Hz : 2H); 3,27 (t dédoublé, J = 7 et 2 Hz : 2H); 3,80 (s : 3H); de 3,85 à 4,00 (mt : 1H); 3,94 (s : 3H); 4,19 (s : 1H); 5,32 (d, J = 8 Hz : 1H); 6,94 (d, J = 9 Hz : 1H); 7,05 (dd, J = 9 et 3 Hz : 1H); 7,23 (mt : 8H); 7,40 (d, J = 3 Hz : 1H)].

### Exemple 10

En opérant selon le mode opératoire de l'exemple 1 mais à partir de 147 mg de chlorure de 3-trifluorométhylphénylsulfonyle, on obtient 8 mg de N-{1-[bis(4-chlorophényl)méthyl]azétidin-3-yl}-3-trifluorométhylphénylsulfonamide sous forme d'une laque de couleur crème [ Spectre de R.M.N. ¹H (300 MHz, CDCl₃, δ en ppm) : 2,79 (t dédoublé, J = 7 et 2 Hz : 2H); 3,41 (t dédoublé, J = 7 et 2 Hz : 2H); 4,03 (mt : 1H); 4,23 (s : 1H); de 4,80 à 5,10 (mf étalé : 1H); de 7,20 à 7,35 (mt : 8H); 7,68 (t, J = 8 Hz : 1H); 7,87 (d large, J = 8 Hz : 1H); 8,05 (d large, J = 8 Hz : 1H); 8,15 (s large : 1H)].

### Exemple 11

En opérant selon le mode opératoire de l'exemple 1 mais à partir de 136 mg de chlorure de napht-2-yl-sulfonyle, on obtient 20 mg de N-{1-[bis(4-chlorophényl)méthyl]azétidin-3-yl}-napht-2-yl-sulfonamide sous forme d'une laque de couleur crème [Spectre de R.M.N. ¹H (400 MHz, CDCl₃, δ en ppm) : 2,74 (mt : 2H); 3,35 (mt : 2H); 4,02 (mt : 1H); 4,17 (s : 1H); 4,96 (mf : 1H); de 7,10 à 7,30 (mt : 8H); 7,64 (mt : 2H); 7,78 (dd, J = 7 et 1,5 Hz : 1H); de 7,90 à 8,05 (mt : 3H); 8,41 (s large : 1H)].

### Exemple 12

En opérant selon le mode opératoire de l'exemple 1 mais à partir de 136 mg de chlorure de napht-1-yl-sulfonyle, on obtient 52 mg de N-{1-[bis(4-chlorophényl)méthyl]azétidin-3-yl}napht-1-yl-sulfonamide sous forme d'une meringue de couleur crème [Spectre de R.M.N. ¹H (300 MHz, CDCl₃, δ en ppm) : 2,63 (t dédoublé, J = 7 et 2 Hz : 2H); 3,20 (t dédoublé, J = 7 et 2 Hz : 2H); 3,90 (mt : 1H); 4,12 (s : 1H); 5,26 (mf : 1H); 7,16 (mt : 8H); 7,52 (t, J = 8 Hz : 1H); de 7,55 à 7,75 (mt : 2H); 7,95 (d, J = 8,5 Hz : 1H); 8,06 (d, J = 8,5 Hz : 1H); 8,23 (dd, J = 7,5 et 1 Hz : 1H); 8,64 (d, J = 8,5 Hz : 1H)].

### Exemple 13

En opérant selon le mode opératoire de l'exemple 1 mais à partir de 128 mg de chlorure de 3,4-difluorophénylsulfonyle, on obtient 7 mg de N-{1-[bis(4-chlorophényl)méthyl]azétidin-3-yl]}-3,4-difluorophénylsulfonamide sous forme d'une laque de couleur crème [Spectre de R.M.N. ¹H (300 MHz, CDCl₃, δ en ppm) : 2,76 (t large, J = 7,5 Hz : 2H); 3,39 (t large, J = 7,5 Hz : 2H); 3,98 (mt : 1H); 4,20 (s large : 1H); de 4,85 à 5,25 (mf étalé : 1H); de 7,15 à 7,35 (mt : 9H); de 7,55 à 7,75 (mt : 2H)].

### Exemple 14

En opérant selon le mode opératoire de l'exemple 1 mais à partir de 108 mg de chlorure de 1-méthylimidazol-4-yl-sulfonyle, on obtient 22 mg de N-{1-[bis(4-chlorophényl)méthyl]azétidin-3-yl}-1-méthyl-1-H-imidazol-4-yl-sulfonamide sous forme d'une meringue de couleur crème [Spectre de R.M.N. ¹H (300 MHz, CDCl₃ avec ajout de quelques gouttes de CD₃COOD d4, δ en ppm) : 3,22 (mt : 2H); 3,67 (mt : 2H); 3,74 (s : 3H); 4,10 (mt : 1H); 4,65 (s large : 1H); 7,27 (mt : 8H); 7,47 (d large, J = 1 Hz : 1H); 7,53 (d large, J = 1Hz : 1H)].

### Exemple 15

En opérant selon le mode opératoire de l'exemple 1 mais à partir de 152 mg de chlorure de 4-acétamido-3-chlorophénylsulfonyle, on obtient 69 mg de N-[4-(N-{1-[bis(4-chlorophényl)méthyl]azétidin-3-yl}sulfamoyl)-2-chlorophényl]acétamide sous forme d'une meringue de couleur crème [Spectre de R.M.N ¹H (300 MHz, CDCl₃, δ en ppm) : 2,30 (s : 3H); 2,73 (mt : 2H); 3,38 (mt : 2H); 3,97 (mt : 1H); 4,19 (s : 1H); 7,24 (s : 8H); 7,70 (dd, J = 7 et 1,5 Hz : 1H); 7,78 (s large : 1H); 7,86 (d, J = 1,5 Hz : 1H); 8,61 (d, J = 7 Hz : 1H)].

### Exemple 16

A une solution de 0,7 g de 1-[bis(4-chlorophényl)méthyl]azétidin-3-ylamine dans 25 cm³ de dichlorométhane on ajoute, à température ambiante sous atmosphère d'argon, 0,79 cm³ de triéthylamine. Le mélange est refroidi vers 0°C, avant d'y ajouter une solution de 1,2 g de chlorure de pyrid-3-yl-sulfonyle dans 25 cm³ de dichlorométhane, puis il est agité à température ambiante pendant 16 heures. Le mélange réactionnel est dilué avec 50 cm³ de dichlorométhane, puis est lavé avec deux fois 25 cm³ d'eau distillée. La phase organique est séchée sur sulfate de magnésium, filtrée et concentrée à sec sous pression réduite (2,7 kPa). Le résidu obtenu est purifié par chromatographie-flash sur gel de silice [éludant: dichlorométhane/méthanol (97,5/2,5 en volumes)]. On obtient 0,7 g de N-{1-[bis(4-chlorophényl)méthyl]azétidin-3-yl}pyrid-3-yl-sulfonamide, sous forme de meringue de couleur crème, qui concrétise en présence d'isopropanol en une poudre crème fondant à 164°C.

Le chlorure de pyrid-3-yl-sulfonyle peut être préparé selon la méthode décrite par Breant, P. et coll., Synthesis, 10, 822-4 (1983).

### Exemple 17

A une solution de 0,307 g de 1-[bis(4-chlorophényl)méthyl]azétidin-3-ylamine dans 10 cm³ de dichlorométhane on ajoute, à température ambiante sous atmosphère d'argon, 0,214 g de chlorure de 4-fluorophénylsulfonyle et 0,28 cm³ de triéthylamine. Après 16 heures d'agitation à température ambiante le mélange réactionnel est lavé avec 10 cm³ d'eau distillée, séché sur sulfate de magnésium, filtré et concentré à sec sous pression réduite (2,7 kPa). Le résidu obtenu est purifié par chromatographie-flash sur gel de silice [éluant : gradient dichlorométhane/acétate d'éthyle (100/0 à 95/5 en volumes)]. On obtient 0,18 g de N-{1-[bis(4-chlorophényl)méthyl]azétidin-3-yl}-4-fluorophénylsulfonamide sous forme de meringue blanche [Spectre de R.M.N. ¹H (300 MHz, CDCl₃, δ en ppm) : 2,74 (t large, J = 7,5 Hz : 2H); 2,39 (t large, J = 7,5 Hz : 2H); 3,98 (mt : 1H); 4,20 (s : 1H); 4,79 (d, J = 9 Hz : 1H); de 7,10 à 7,35 (mt : 10H); 7,86 (mt : 2H)].

### Exemple 18

En opérant selon le mode opératoire de l'exemple 17 mais à partir de 0,25 g de chlorure de quinol-8-ylsulfonyle, on obtient 0,36 g de N-{1-[bis(4-chlorophényl)méthyl]azétidin-3-yl}quinol-8-ylsulfonamide sous forme d'une poudre blanche [Spectre de R.M.N. ¹H-(300 MHz, CDCl₃, δ en ppm) : 2,63 (t dédoublé, J = 7 et 2 Hz : 2H); 3,16 (t dédoublé, J = 7 et 2 Hz : 2H); 3,98 (mt : 1H); 4,11 (s : 1H); 6,77 (d, J = 8 Hz : 1H); 7,15 (mt : 8H); 7,61 (dd, J = 8 et 4 Hz : 1H); 7,64 (dd, J = 8 et 7,5 Hz : 1H); 8,06 (dd, J = 8 et 1,5 Hz : 1H); 8,30 (dd, J = 8 et 1,5 Hz : 1H); 8,40 (dd, J = 7,5 et 1,5 Hz : 1H); 9,09 (dd, J = 4 et 1,5 Hz : 1H)].

### Exemple 19

En opérant selon le mode opératoire de l'exemple 17 mais à partir de 0,14 cm³ de chlorure de phénylsulfonyle, on obtient 0,35 g de N-{1-[bis(4-chlorophényl)méthyl]azétidin-3-yl}phénylsulfonamide sous forme d'une poudre blanche [Spectre de R.M.N. ¹H (300 MHz, CDCl₃, δ en ppm) : 2,75 (t large, J = 7,5 Hz : 2H); 3,40 (t large, J = 7,5 Hz : 2H); 4,03 (mt : 1H); 4,22 (s : 1H); 4,79 (d, J = 10 Hz : 1H); 7,31 (s : 8H); de 7,45 à 7,65 (mt : 3H); 7,87 (d large, J = 7,5 Hz : 2H)].

### Exemple 20

En opérant selon le mode opératoire de l'exemple 17 mais à partir de 0,21 g de chlorure de (phénylméthyl)sulfonyle, on obtient 0,27 g de N-{1-[bis(4-chlorophényl)méthyl]azétidin-3-yl}-(phénylméthyl)sulfonamide sous forme d'une poudre blanche [Spectre de R.M.N. ¹H (400 MHz, CDCl₃, δ en ppm) :: 2,76 (t dédoublé, J = 7 et 2 Hz : 2H); 3,41 (t dédoublé, J = 7 et 2 Hz : 2H); 3,85 (mt : 1H); 4,20 (s : 1H); 4,23 (s : 2H); 4,46 (d, J = 9 Hz : 1H); de 7,25 à 7,45 (mt: 13H)].

### Exemple 21

En opérant selon le mode opératoire de l'exemple 17 mais à partir de 0,42 g de chlorure de 3,5-difluorophénylsulfonyle dans 30 cm³ de dichlorométhane et en lavant la phase organique par deux fois 20 cm³ d'eau distillée. Après purification par chromatographie-flash sur gel de silice [éluant : gradient dichlorométhane/méthanol (100/0 à 95/5 en volumes)] on obtient 0,1 g de N-{1-[bis(4-chlorophényl)méthyl]azétidin-3-yl}-3,5-difluorophénylsulfonamide sous forme d'une poudre jaune [Spectre de R.M.N. ¹H (300 MHz, CDCl₃, δ en ppm) : 2,77 (t dédoublé, J = 7 et 2 Hz : 2H); 3,41 (t dédoublé, J = 7 et 2 Hz : 2H); 4,01 (mt : 1H); 4,21 (s : 1H); 4,90 (d, J = 9 Hz : 1H); 7,02 (tt, J = 8,5 et 2,5 Hz : 1H); de 7,20 à 7,35 (mt : 8H); 7,38 (mt : 2H) ).

Le chlorure de 3,5-difluorophénylsulfonyle peut être préparé selon la méthode décrite dans le brevet FR 9615887.

### Exemple 22

En opérant selon le mode opératoire de l'exemple 21 mais à partir de 0,21 g de chlorure de pyrid-2-ylsulfonyle et de 0,17 cm³ de triéthylamine et en lavant la phase organique par deux fois 30 cm³ d'eau distillée. Après purification par chromatographie-flash sur gel de silice [éluant : gradient dichlorométhane/méthanol (100/0 à 98/2 en volumes)] on obtient, 0,3 g de N-{1-[bis(4-chlorophényl)méthyl]azétidin-3-yl}pyrid-2-ylsulfonamide sous forme d'une poudre blanche [Spectre de R.M.N. ¹H (300 MHz, CDCl₃, δ en ppm) : 2,78 (t dédoublé, J = 7 et 2 Hz : 2H); 3,35 (t dédoublé, J = 7 et 2 Hz : 2H); 4,12 (mt : 1H); 4,20 (s : 1H); 5,30 (d, J = 9 Hz : 1H); de 7,15 à 7,35 (mt : 8H); 7,47 (ddd, J = 7,5 et 5 et 1 Hz : 1H); 7,90 (t dédoublé, J = 7,5 et 2 Hz : 1H); 7,98 (d large, J = 7,5 Hz : 1H); 8,65 (d large, J = 5 Hz : 1H)].

Le chlorure de pyrid-2-ylsulfonyle peut être préparé selon la méthode décrite par Corey, E. J. et coll., J. Org. Chem. (1989), 54(2), 389-93.

### Exemple 23

A 0,24 g de N-{1-[bis(4-chlorophényl)méthyl]azétidin-3-yl}-(3,5-difluorophényl)sulfonamide en solution dans 6 cm³ de diméthylsulfoxyde on ajoute, à température ambiante, 0,104 cm³ de pyrrolidine, puis le mélange est chauffé 18 heures à 90°C. Le mélange réactionnel est dilué avec 30 cm³ de dichlorométhane et lavé avec trois fois 30 cm³ d'eau distillée. La phase organique est séchée sur sulfate de magnésium, filtrée et concentrée à sec sous pression réduite (2,7 kPa). Le résidu est purifié par chromatographie-flash sur gel de silice en éluant avec du dichlorométhane. On obtient ainsi 50 mg de N-{1-[bis(4-chlorophényl)méthyl]azétidin-3-yl}-(3-fluoro-5-pyrrolidin-1-yl-phényl)sulfonamide sous forme d'une poudre blanche [Spectre de R.M.N. ¹H (600 MHz, CDCl₃ avec ajout de quelques gouttes de CD₃COOD d4, δ en ppm); 2,04 (mt : 4H); de 3,20 à 3,35 (mt : 6H); 3,60 (t, J = 8,5 Hz : 2H); 4,14 (mt: 1H); 4,57 (s: 1H); 6,31 (d large, J = 11,5 Hz : 1H); 6,70 (d large, J = 8,5 Hz : 1H); 6,72 (s large : 1H); de 7,20 à 7,35 (mt : 8H)].

### Exemple 24

A une suspension de 20,5 mg d'hydrure de sodium à 80% dans 10 cm³ de tétrahydrofuranne on ajoute, à température ambiante sous atmosphère d'argon, une solution de 0,26 g de N-{1-[bis(4-chlorophényl)méthyl]azétidin-3-yl}-4-fluorophénylsulfonamide dans 5 cm³ de tétrahydrofuranne. Après 1 heure d'agitation vers 20°C on ajoute 60 mm³ d'iodométhane, puis après 16 heures d'agitation supplémentaires la suspension est additionnée de 30 cm³ d'acétate d'éthyle et 20 cm³ d'eau distillée. La phase organique est séchée sur sulfate de magnésium, filtrée et concentrée à sec sous pression réduite (2,7 kPa). Le résidu est purifié par chromatographie-flash sur gel de silice [éluant : cyclohexane/acétate d'éthyle (90/10 en volumes)]. On obtient ainsi 19 mg de N-{1-[bis(4-chlorophényl)méthyl]azétidin-3-yl}-N-méthyl-4-fluorophénylsulfonamide sous forme d'une poudre blanche [Spectre de R.M.N ¹H (300 MHz, CDCl₃, δ en ppm) : 2,69 (s : 3H); 3,02 (t dédoublé, J = 7 et 2 Hz : 2H); 3,35 (t dédoublé, J = 7 et 2 Hz : 2H); 3,91 (mt : 1H); 4,27 (s : 1H); de 7,15 à 7,35 (mt : 10H); 7,75 (dd, J = 9 et 5 Hz : 2H)].

### Exemple 25

En opérant selon le mode opératoire de l'exemple 24 mais à partir de 0,25 g de N-{1-[bis(4-chlorophényl)méthyl]azétidin-3-yl}quinol-8-ylsulfonamide et de 18 mg d'hydrure de sodium à 80%. Après purification par chromatographie-flash sur gel de silice [éluant : cyclohexane/acétate d'éthyle (80/20 en volumes)], on obtient 70 mg de N-{1-[bis(4-chlorophényl)méthyl]azétidin-3-yl}-N-méthyl-quinol-8-ylsulfonamide [Spectre de R.M.N ¹H (300 MHz, CDCl₃, δ en ppm) : de 3,00 à 3,10 (mt : 2H); 3,05 (s : 3H); 3,35 (mt : 2H); 4,27 (s : 1H); 4,93 (mt : 1H); de 7,15 à 7,35 (mt : 8H); 7,50 (dd, J = 8,5 et 4 Hz : 1H); 7,62 (dd, J = 8 et 8,5 Hz : 1H); 8,03 (dd, J = 8,5 et 1,5 Hz : 1H); 8,22 (dd, J = 8,5 et 1,5 Hz : 1H); 8,48 (dd, J = 8 et 1,5 Hz : 1H); 8,98 (dd, J = 4 et 1,5 Hz : 1H)].

### Exemple 26

En opérant selon le mode opératoire de l'exemple 24 mais à partir de 0,21 g de N-{1-[bis(4-chlorophényl)méthyl]azétidin-3-yl}phénylsulfonamide, de 17 mg d'hydrure de sodium à 80% et en introduisant l'iodométhane en deux fois à 3 heures d'intervalle. On obtient ainsi 80 mg de N-{1-[bis(4-chlorophényl)méthyl]azétidin-3-yl}-N-méthyl-phénylsulfonamide sous forme de laque blanche [Spectre de R.M.N ¹H (300 MHz, CDCl₃, δ en ppm) : 2,70 (s : 3H); 3,03 (t large, J = 7,5 Hz : 2H); 3,37 (t large, J = 7,5 Hz : 2H); 3,94 (mt : 1H); 4,28 (s : 1H); de 7,20 à 7,35 (mt : 8H); de 7,45 à 7,65 (mt : 3H); 7,74 (d large, J = 8 Hz : 2H)].

### Exemple 27

En opérant selon le mode opératoire de l'exemple 26 mais à partir de 0,17 g de N-{1-[bis(4-chlorophényl)méthyl]azétidin-3-yl}-(phénylméthyl)sulfonamide, de 14 mg d'hydrure de sodium à 80% et en laissant agiter 48 heures à 20°C. Après purification par chromatographie-flash sur gel de silice [éluant : dichlorométhane/acétate d'éthyle (95/5 en volumes)], on obtient 120 mg de N-{1-[bis(4-chlorophényl)méthyl]azétidin-3-yl}-N-méthyl-(phénylméthyl)sulfonamide sous forme d'une meringue blanche [Spectre de R.M.N ¹H (300 MHz, CDCl₃, δ en ppm) : 2,81 (s : 3H); 2,88 (t dédoublé, J = 7 et 2 Hz : 2H); 3,16 (t dédoublé, J = 7 et 2 Hz : 2H); de 4,10 à 4,25 (mt : 4H); de 7,20 à 7,40 (mt: 13H)].

### Exemple 28

A une solution de 0,412 g du dichlorure de l'acide benzène-1,3-disulfonique et de 0,165 cm³ de triéthylamine dans 20 cm³ d'acétonitrile, on ajoute goutte à goutte, à température ambiante une solution de 0,307 g 1-[bis(4-chlorophényl)méthyl]azétidin-3-ylamine dans 10 cm³ d'acétonitrile. Après 3 heures d'agitation à température ambiante, 0,28 cm³ d'une solution à 20% d'ammoniac est ajoutée et le mélange réactionnel laissé à température ambiante Après 18 heures, le mélange est filtré et concentré à sec sous pression réduite (2,7 kPa). Après chromatographie sur une colonne de gel de silice (granulométrie 0,06-0,200 mm, hauteur 35 cm, diamètre 2 cm), en éluant sous une pression de 0,9 bar d'argon avec du dichlorométhane puis un mélange de dichlorométhane + 1% de méthanol puis un mélange de dichlorométhane + 2% de méthanol en volume et en recueillant des fractions de 30 cm³, les fractions 23 à 34 sont réunies et concentrées à sec sous pression réduite (2,7 kPa) pour obtenir 90 mg de N-{1-[bis(4-chlorophényl)méthyl]azétidin-3-yl}-3-sulfamoylphénylsulfonamide sous la forme d'un solide blanc [Spectre de R.M.N. ¹H (300 MHz, CDCl₃, δ en ppm) : 2,78 (t large, J = 7 Hz : 2H); 3,35 (t large, J = 7 Hz : 2H); 4,01 (mt : 1H); 4,24 (s : 1H); 5,27 (mf : 2H); 5,61 (mf : 1H); de 7,15 à 7,35 (mt : 8H); 7,67 (t, J = 8 Hz : 1H); 8,04 (d large, J = 8 Hz : 1H); 8,12 (d large, J = 8 Hz : 1H); 8,49 (s large : 1H)].

### Exemple 29

A une solution de 80,1 mg d'acide benzènesulfonyl acétique, 27 mg d'hydroxybenzotriazole en solution dans 0,5 cm³ de diméthylformamide sous atmosphère inerte d'argon, à une température voisine de 23°C, sont ajoutés 0,031 cm³ de diisopropylcarbodiimide, une solution de 30 mg de 1-[bis(4-chlorophényl)méthyl]azétidin-3-yl-amine dans 0,5 cm³ de dichlorométhane anhydre, et 3 cm³ de dichlorométhane anhydre. Après 17 heures à une température voisine de 23°C, le mélange réactionnel est chargé sur une cartouche SPE de 3 cm³ contenant 1 g de phase SCX préconditionnée avec du méthanol. Après lavages avec 2 fois 5 cm³ de méthanol, puis 4 cm³ de méthanol ammoniacal 0,1N, le produit attendu est élué avec 4 cm³ de méthanol ammoniacal 1N. La fraction contenant le produit attendu est évaporée sous flux d'air à une température voisine de 45°C, puis séchée sous pression réduite (1 mbar) à une température voisine de 40°C. On obtient ainsi le 2-benzènesulfonyl-N-{1-[bis-(4-chlorophényl)-méthyl]-azétidin-3-yl}-acétamide sous forme d'un solide blanc [Spectre de R.M.N. ¹H (500 MHz, CDCl₃, δ en ppm) : 2,96 (mt : 2H); 3,51 (mt : 2H); 4,00 (s : 2H); 4,34 (mf : 1H); 4,48 (mt : 1H); 7,10 (mf : 1H); de 7,20 à 7,45 (mt : 8H); 7,57 (t, J = 8 Hz : 2H); 7,70 (t, J = 8 Hz : 1H); 7,90 (d, J = 8 Hz : 2H)].

### Exemple 30

A une solution de 85,7 mg d'acide toluènesulfonyl acétique, 27 mg d'hydroxybenzotriazole en solution dans 0,5 cm³ de diméthylformamide sous atmosphère inerte d'argon, à une température voisine de 23°C, sont ajoutés 0,031 cm³ de diisopropylcarbodiimide, une solution de 30 mg de 1-[bis(4-chlorophényl)méthyl]azétidin-3-yl-amine dans 0,5 cm³ de dichlorométhane anhydre, et 3 cm³ de dichlorométhane anhydre. Après 17 heures à une température voisine de 23°C, le mélange réactionnel est chargé sur une cartouche SPE de 3 cm³ contenant 1 g de phase SCX préconditionnée avec du méthanol. Après lavages avec 2 fois 5 cm³ de méthanol, puis 4 cm³ de méthanol ammoniacal 0,1N, le produit attendu est élué avec 4 cm³ de méthanol ammoniacal 1N. La fraction contenant le produit attendu est évaporée sous flux d'air à une température voisine de 45°C, puis séchée sous pression réduite (1 mbar) à une température voisine de 40°C. On obtient le N-{1-[bis-(4-chlorophényl)-méthyl]-azétidin-3-yl}-2-(toluène-4-sulfonyl)-acétamide sous forme d'une laque jaune [Spectre de R.M.N. ¹H (500 MHz, CDCl₃, δ en ppm) : 2,85 (t, J = 7 Hz : 2H); 3,07 (s :3H); 3,48 (t, J = 7 Hz : 2H); 4,24 (s : 1H); 4,49 (mt : 1H); 7,19 (d large, J = 6 Hz : 1H); de 7,20 à 7,40 (mt : 8H); 8,40 (s : 1H]).

### Exemple 31

En opérant selon le mode opératoire de l'exemple 30, à partir de 85,7 mg d'acide 3-chloro-4-méthylsulfony-thiophène-2-carboxylique, 27 mg d'hydroxybenzotriazole en solution dans 0,5 cm³ de diméthylformamide, 0,031 cm³ de diisopropylcarbodiimide, une solution de 30 mg de 1-[bis(4-chlorophényl)méthyl]azétidin-3-yl-amine dans 0,5 cm³ de dichlorométhane anhydre et 3 cm³ de dichlorométhane anhydre, on obtient le (3-chloro-4-méthylsulfonyl-thiophène-2-carboxy)-{1-[bis-(4-chlorophényl)-méthyl]-azétidin-3-yl}-amide sous forme d'une laque jaune [Spectre de R.M.N. ¹H (500 MHz, CDCl₃, δ en ppm) : 2,44 (s : 3H); 2,96 (mf : 2H); 3,52 (mf : 2H); 3,98 (s : 2H); 4,35 (mf : 1H); 4,49 (mt : 1H); de 7,00 à 7,30 (mf étalé : 1H); de 7,20 à 7,45 (mt : 10H); 7,76 (d, J = 8 Hz : 2H)].

### Exemple 32

En opérant selon le mode opératoire de l'exemple 30, à partir de 96,1 mg d'acide 3-(2-phényl-éthylènesulfonyl)-propionique, 27 mg d'hydroxybenzotriazole en solution dans 0,5 cm³ de diméthylformamide, 0,031 cm³ de diisopropylcarbodiimide, une solution de 30 mg de 1-[bis(4-chlorophényl)méthyl]azétidin-3-yl-amine dans 0,5 cm³ de dichlorométhane anhydre, et 3 cm³ de dichlorométhane anhydre, on obtient le N-{1-[bis-(4-chlorophényl)-méthyl]-azétidin-3-yl}-3-(2-phényl-éthylènesulfonyl)-propionamide sous forme d'une meringue blanche [Spectre de R.M.N. ¹H (500 MHz, CDCl₃, δ en ppm) : 2,64 (t, J = 7 Hz : 2H); 2,88 (mf : 2H); 3,33 (t,

J = 7 Hz : 2H); 3,49 (mf : 2H); 4,29 (mf : 1H); 4,48 (mt : 1H); de 5,90 à 6,15 (mf étalé : 1H); 6,41 (d, J = 12 Hz : 1H); 7,17 (d, J = 12 Hz : 1H); de 7,20 à 7,35 (mt : 8H); 7,41 (mt : 3H); 7,64 (mt : 2H)].

### Exemple 33

En opérant selon le mode opératoire de l'exemple 31, à partir de 58,5 mg d'acide 4-méthylsulfonyl-benzoïque, 26,4 mg d'hydroxybenzotriazole en solution dans 0,5 cm³ de diméthylformamide, 0,0302 cm³ de diisopropylcarbodiimide, une solution de 30 mg de 1-[bis(4-chlorophényl)méthyl]azétidin-3-yl-amine dans 0,5 cm³ de dichlorométhane anhydre, et 3 cm³ de dichlorométhane anhydre, on obtient le N-{1-[bis-(4-chlorophényl)-méthyl]-azétidin-3-yl}-4-méthylsulfonyl-benzamide sous forme d'une cristaux blanc [Spectre de R.M.N ¹H (300 MHz, CDCl₃, δ en ppm) : 3,03 (mt : 2H); 3,09 (s : 3H); 3,61 (t large, J = 7,5 Hz : 2H); 4,35 (s : 1H); 4,73 (mt : 1H); 6,55 (d large, J = 7,5 Hz : 1H); de 7,20 à 7,35 (mt : 8H); 7,96 (d, J = 8 Hz : 2H); 8,03 (d, J = 8 Hz : 2H)].

### Exemple 34

En opérant selon le mode opératoire de l'exemple 31, à partir de 58,5 mg d'acide 3-phénylsulfonyl-propionique, 26,4 mg d'hydroxybenzotriazole en solution dans 0,5 cm³ de diméthylformamide, 0,0302 cm³ de diisopropylcarbodiimide, une solution de 30 mg de 1-[bis(4-chlorophényl)méthyl]azétidin-3-yl-amine dans 0,5 cm³ de dichlorométhane anhydre, et 3 cm³ de dichlorométhane anhydre, on obtient le N-{1-[bis-(4-chlorophényl)-méthyl]-azétidin-3-yl}-4-méthanesulfonyl-benzamide sous forme d'une laque [Spectre de R.M.N ¹H (300 MHz, CDCl₃, δ en ppm) : 2,71 (t, J = 7,5 Hz : 2H); 2,86 (mt : 2H); de 3,40 à 3,55 (mt : 4H); 4,26 (s : 1H); 4,45 (mt : 1H); 6,22 (d large, J = 7,5 Hz : 1H); de 7,20 à 7,35 (mt : 8H); 7,59 (t large, J = 7,5 Hz : 2H); 7,69 (tt, J = 7,5 et 1,5 Hz : 1H); 7,93 (d large, J = 7,5 Hz : 2H)].

### Exemple 35

En opérant selon le mode opératoire de l'exemple 31, à partir de 60,2 mg d'acide 5-méthylsulfonyl-thiophène-2-carboxylique, 26,4 mg d'hydroxybenzotriazole en solution dans 0,5 cm³ de diméthylformamide, 0,0302 cm³ de diisopropylcarbodiimide, une solution de 30 mg de 1-[bis(4-chlorophényl)méthyl]azétidin-3-yl-amine dans 0,5 cm³ de dichlorométhane anhydre, et 3 cm³ de dichlorométhane anhydre, on obtient le (5-méthylsulfonyl-thiophène-2-carboxy)-{1-[bis-(4-chlorophényl)-méthyl]-azétidin-3-yl}-amide sous forme de cristaux blancs [Spectre de R.M.N ¹H (300 MHz, CDCl₃, δ en ppm) : 3,03 (mt : 2H); 3,21 (s : 3H); 3,57 (dd, J = 8 et 7,5 Hz : 2H); 4,34 (s : 1H); 4,67 (mt : 1H); 6,40 (d large, J = 7,5 Hz : 1H); de 7,20 à 7,35 (mt : 8H); 7,48 (d, J = 4 Hz : 1H); 7,67 (d, J = 4 Hz : 1H)].

### Exemple 36

En opérant selon le mode opératoire de l'exemple 31, à partir de 71,9 mg d'acide 5-méthylsulfonyl-3-méthyl-4-vinyl-thiophène-2-carboxylique, 26,4 mg d'hydroxybenzotriazole en solution dans 0,5 cm³ de diméthylformamide, 0,0302 cm³ de diisopropylcarbodiimide, une solution de 30 mg de 1-[bis(4-chlorophényl)méthyl]azétidin-3-yl-amine dans 0,5 cm³ de dichlorométhane anhydre, et 3 cm³ de dichlorométhane anhydre, on obtient le (5-méthylsulfonyl-3-méthyl-4-vinyl-thiophène-2-carboxy)-{1-[bis-(4-chlorophényl)-méthyl]-azétidin-3-yl}-amide sous forme d'une poudre blanche [Spectre de R.M.N ¹H (300 MHz, CDCl₃, δ en ppm) : 2,47 (s : 3H); 2,97 (mt : 2H); 3,14 (s : 3H); 3,57 (dd, J = 8 et 7,5 Hz : 2H); 4,32 (s : 1H); 4,65 (mt : 1H); 5,69 (dd, J = 18 et 1 Hz : 1H); 5,77 (dd, J = 12 et 1 Hz : 1H); 6,30 (d large, J = 7,5 Hz : 1H); 6,96 (dd, J = 18 et 12 Hz : 1H); de 7,20 à 7,35 (mt : 8H)].

### Exemple 37

En opérant selon le mode opératoire de l'exemple 31, à partir de 62,6 mg d'acide 3-méthylsulfonylméthyl-benzoïque, 26,4 mg d'hydroxybenzotriazole en solution dans 0,5 cm³ de diméthylformamide, 0,0302 cm³ de diisopropylcarbodiimide, une solution de 30 mg de 1-[bis(4-chlorophényl)méthyl]azétidin-3-yl-amine dans 0,5 cm³ de dichlorométhane anhydre, et 3 cm³ de dichlorométhane anhydre, on obtient le (5-méthylsulfonyl-3-méthyl-4-vinyl-thiophène-2-carboxy)-{1-[bis-(4-chlorophényl)-méthyl]-azétidin-3-yl}-amide sous forme d'aiguilles blanches [Spectre de R.M.N ¹H (300 MHz, (CD₃)₂SO d6 avec ajout de CDCl₃, δ en ppm) : 2,84 (s : 3H); 3,02 (t large, J = 7 Hz : 2H); 3,48 (t, J = 7 Hz : 2H); 4,38 (s : 3H); 4,53 (mt : 1H); 7,21 (d, J = 8 Hz : 4H); 7,34 (d, J = 8 Hz : 4H); 7,40 (t, J = 7,5 Hz : 1H); 7,53 (d large, J = 7,5 Hz : 1H); 7,84 (d large, J = 7,5 Hz : 1H); 7,89 (s large : 1H); 8,54 (d, J = 7 Hz : 1H)].

### Exemple 38

Le (RS)-N-{1-[(4-chloro-phényl)-pyridin-3-yl-méthyl]-azétidin-3-yl}-3,5-difluorobenzènesulfonamide peut être obtenu de la façon suivante : A un mélange de 0,3 g de bromhydrate de (RS)-3-[bromo-(4-chlorophényl)-méthyl]-pyridine et de 0,28 g de chlorhydrate de N-azétidin-3-yl-3,5-difluoro-benzenesulfonamide dans 20 cm³ d'acétonitrile on ajoute 0,46 g de carbonate de potassium et 41 mg de iodure de potassium puis on chauffe le mélange à reflux pendant 4 heures. Après refroidissement à une température voisine de 20°C, on élimine les matières insolubles par filtration puis on concentre à sec sous pression réduite. Le résidu obtenu est repris avec 100 cm³ d'acétate d'éthyle. La phase organique est lavée avec 2 fois 50 cm³ d'eau, séchée sur sulfate de magnésium en présence de noir animal, filtrée sur Célite, puis concentrée à sec sous pression réduite. On obtient 230 mg d'un solide orange qui est dissout dans un mélange cyclohexane - acétate d'éthyle (mélange 50 - 50 en volumes) et purifié par chromatographie sous pression sur une cartouche de 10 g de silice avec le même mélange éluant, avec un débit de 6 cm³/minute. Les fractions 22 à 56 sont rassemblées, concentrées à sec sous pression réduite. On obtient ainsi 100 mg de (RS)-N-{1-[(4-chloro-phényl)-pyridin-3-yl-méthyl]-azétidin-3-yl}-3,5-difluoro-benzenesulfonamide sous forme d'une meringue jaune pâle fondant à 70°C [Spectre de R.M.N. ¹H (300 MHz, CDCl₃, 8 en ppm) : 2,81 (mt : 2H); 3,42 (mt : 2H); 4,03 (mt : 1H); 4,29 (s : 1H ); 5,43 (d, J = 9 Hz : 1H); 7,01 (tt, J = 9 et 2,5 Hz : 1H); 7,22 (dd, J = 8 et 5 Hz : 1H); 7,28 (mt : 4H); 7,36 (mt : 2H); 7,62 (d large, J = 8 Hz : 1H); 8,48 (dd, J = 5 et 1 Hz : 1H); 8,59 (d, J = 1 Hz : 1H)].

La (RS)-3-[bromo-(4-chlorophényl)-méthyl]-pyridine est obtenue de la façon suivante : A 1,5g de (4-chlorophényl)-pyridin-3-yl-methanol on ajoute 3,5 cm³ d'une solution d'acide bromhydrique à 48% dans l'acide acétique et 1 cm³ de bromure d'acétyle. Le mélange de couleur ambrée ainsi obtenu est chauffé au reflux pendant 4 heures puis refroidi à 20°C, concentré à sec à 40°C sous 2,7 kPa conduisant à 1,53g de (RS)-3-[bromo-(4-chlorophényl)-méthyl]-pyridine (Rf=75/90, 254nm, Plaques de Silice, référence 1.05719, Merck KGaA, 64271 Darmstatd, Allemagne).

Le chlorhydrate de N-azétidin-3-yl-3,5-difluoro-benzenesulfonamide peut être préparé de la manière suivante : Dans un hydrogénateur de 2000 cm³, une solution de 7,5 g de N-(1-benzhydryl-azétidin-3-yl)-3,5-difluorobenzenesulfonamide dans un mélange de 10 cm³ d'acide chlorhydrique concentré (36% en poids), de 1,7 cm³ d'acide acétique et de 500 cm³ de méthanol est hydrogénée en présence de 4,21 g d'hydroxyde de palladium sur charbon (20% en poids de catalyseur) sous 1,7 bars d'hydrogène pendant environ 20 heures. Le catalyseur est éliminé par filtration sur un lit de célite puis le filtrat est concentré à sec sous pression réduite. Le résidu obtenu est battu avec 100 cm³ d'éther diisopropylique pendant environ 16 heures à une température voisine de 20°C. La suspension est filtrée, et le résidu solide est de nouveau battu avec 100 cm³ d'éther diéthylique à une température voisine de 20°C. Après filtration, la pâte obtenue est séchée sous pression réduite à une température voisine de 40°C. On obtient ainsi 5,52 g de chlorhydrate de N-azétidin-3-yl-3,5-difluoro-benzenesulfonamide sous forme d'une poudre blanche.

Le N-(1-benzhydryl-azétidin-3-yl)-3,5-difluoro-benzenesulfonamide peut être préparé de la manière suivante : A une suspension de 5 g de 1-benzhydrylazétidin-3-ylamine dans 80 cm³ de dichlorométhane, à une température voisine de 20°C, sont ajoutés successivement 5,1 g de chlorure de 3,5-difluoro-benzenesulfonyle puis 4,2 cm³ de triéthylamine. Après 20 heures d'agitation à une température voisine de 20°C, on ajoute 50 cm³ d'eau. La phase organique décantée est lavée avec 2 fois 50 cm³ d'eau, séchée sur sulfate de magnésium, et concentrée à sec sous pression réduite. On obtient ainsi 8,99 g d'une huile jaune qui cristallise peu à peu. 4,5 g de ce produit est purifié par chromatographie sous pression sur 500 g de silice Amicon (diamètre des particules de 0,020 à 0,045 mm) en éluant avec un mélange méthanol - dichlorométhane (1 - 99 en volumes). Les fractions contenant le produit recherché sont rassemblées et concentrées à sec sous pression réduite pour fournir 3,58 g de N-(1-benzhydryl-azétidin-3-yl)-3,5-difluorobenzenesulfonamide sous forme d'une poudre beige. La quantité restante de l'huile jaune précédente est purifiée dans les mêmes conditions et fournit 3,92 g de N-(1-benzhydryl-azétidin-3-yl)-3,5-difluoro-benzenesulfonamide sous forme d'une poudre beige.

La 1-benzhydryl-azétidin-3-ylamine peut être préparée comme décrit dans J. Antibiot., 39(9), 1243-1256, 1986.

Le chlorure de 3,5-difluoro-benzenesulfonyle peut être préparé comme décrit dans le brevet : FR2757509.

### Exemple 39

Le (RS)-N-{1-[(4-chloro-phényl)-pyrimidin-5-yl-méthyl]-azétidin-3-yl}-3,5-difluoro-benzenesulfonamide peut être obtenu en opérant comme pour la préparation du (RS)-N-{1-[(4-chloro-phényl)-pyridin-3-yl-méthyl]-azétidin-3-yl}-3,5-difluoro-benzenesulfonamide : à partir de 0,64 g de bromhydrate de (RS)-5-[bromo-(4-chloro-phényl)-méthyl]-pyrimidine, de 0,5 g de chlorhydrate de N-azétidin-3-yl-3,5-difluoro-benzenesulfonamide dans 20 cm³ d'acétonitrile, de 1,213 g de carbonate de potassium et de 379 mg de iodure de potassium, on obtient ainsi 71 mg de (RS)-N-{1-[(4-chloro-phényl)-pyrimidin-5-yl-méthyl]-azétidin-3-yl}-3,5-difluoro-benzenesulfonamide sous forme d'une meringue jaune [Spectre de R.M.N. ¹H (300 MHz, CDCl₃, δ en ppm) : 2,83 (mt : 2H); 3,46 (mt : 2H); 4,03 (mt : 1H); 4,30 (s : 1H); 5,00 (d, J = 9 Hz : 1H); 7,04 (tt, J = 9 et 2,5 Hz : 1H); de 7,20 à 7,35 (mt : 4H); 7,37 (mt : 2H); 8,69 (s : 2H); 9,09 (s : 1H)].

La (RS)-5-[bromo-(4-chloro-phényl)-méthyl]-pyrimidine peut être obtenue en opérant comme pour la préparation de la (RS)-3-[bromo-(4-chlorophényl)-méthyl]-pyridine en utilisant comme matière première le (4-chloro-phényl)-pyrimidin-5-yl-méthanol.

Le (4-chloro-phényl)-pyrimidin-5-yl-méthanol peut être préparé en opérant comme pour le (4-chlorophényl)-pyridin-3-yl-méthanol, à partir de pyrimidin-5-carboxaldéhyde et de bromure de 4-chlorophényl magnésium.

Les médicaments selon l'invention sont constitués par un composé de formule (I) ou un isomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops ets élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des psychoses y compris la schizophrénie, des troubles anxieux, de la dépression, de l'épilepsie, de la neurodégénération, des désordres cérébelleux et spinocérébelleux, des désordres cognitifs, du trauma crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, des troubles du transit intestinal, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques,.

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc . 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml
et azote et étant éventuellement substitué par un ou plusieurs alkyle, oxo ou -CO-NH₂,

R₁₂ et R₁₃, identiques ou différents, représentent un atome d'hydrogène ou un radical alkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,

R₁₄ et R₁₅, identiques ou différents, représentent un atome d'hydrogène ou un radical alkyle, cycloalkyle, alkylcycloalkyle ou hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, oxo, hydroxyalkyle ou -CO-NH₂,

Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, vinyle, halogène, alcoxycarbonyle, oxo, hydroxy, les hétérocycles azotés étant éventuellement sous leur forme N-oxydée et, de préférence, Het représente un hétérocycle choisi parmi les hétérocycles suivants : benzimidazole, benzoxazole, benzothiazole, benzothiophène, thiophène, quinazoline, quinoxaline, quinoline, pyrrole, pyridine, imidazole, indole, isoquinoline, pyrimidine, thiazole, thiadiazole, furane, tétrahydroisoquinoline et tétrahydroquinoline, ces hétérocycles étant éventuellement substitués par un ou plusieurs alkyle, alcoxy, vinyle, halogène, oxo, hydroxy, OCF₃ ou CF₃,
leurs isomères optiques et leurs sels pharmaceutiquement acceptables,
à l'exception du composé pour lequel R₂ et R₃ représentent des radicaux phényle, R₁ représente un radical -N(R₅)-Y-R₆, Y est SO₂, R₅ représente un radical méthyle et R₆ représente un radical phényle.

8 - Composés de formule (I) selon la revendication 5 dans laquelle
R₁ représente un radical -N(R₅)-Y-R₆,
Y est SO₂,
R₂ représente soit un phényle non substitué ou substitué par un ou plusieurs halogène, alkyle, alcoxy, trifluorométhyle, trifluorométhoxy ou hydroxyalkyle; soit un hétéroaromatique choisi parmi les cycles pyridyle et pyrimidyle, ces hétéroaromatiques pouvant être non substitués ou substitués par un halogène, alkyle, alcoxy, hydroxy, trifluorométhyle ou trifluorométhoxy,
R₃ représente soit un phényle non substitué ou substitué par un ou plusieurs halogène, alkyle, alcoxy, trifluorométhyle, trifluorométhoxy, hydroxyalkyle; soit un hétéroaromatique choisi parmi les cycles pyridyle et pyrimidyle, ces hétéroaromatiques pouvant être non substitués ou substitués par un halogène, alkyle, alcoxy, hydroxy, trifluorométhyle ou trifluorométhoxy,
R₅ représente un atome d'hydrogène ou alkyle,
R₆ représente un radical naphtyle, phénylalkyle, Het ou phényle éventuellement substitué par un ou plusieurs halogène, alkyle, alcoxy, -NR₁₄R₁₅, hydroxy, hydroxyalkyle, OCF₃, CF₃ ou -SO₂NH₂, ou bien sur 2 atomes de carbone adjacents par dioxyméthylène
R₁₄ et R₁₅, identiques ou différents représentent un atome d'hydrogène ou un radical alkyle, cycloalkyle, alkylcycloalkyle ou hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, oxo, hydroxyalkyle ou -CO-NH₂,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, vinyle, halogène, alcoxycarbonyle, oxo, hydroxy, les hétérocycles azotés étant éventuellement sous leur forme N-oxydée et, de préférence, Het représente un hétérocycle choisi parmi les hétérocycles suivants : benzimidazole, benzoxazole, benzothiazole, benzothiophène, thiophène, quinoline, pyrrole, pyridine, pyrimidine, thiazole, thiadiazole, furane, tétrahydroisoquinoline, tétrahydroquinoline, ces hétérocycles étant éventuellement substitués par un ou plusieurs alkyle, alcoxy, vinyle, halogène, oxo, hydroxy, OCF₃ ou CF₃,
leurs isomères optiques et leurs sels pharmaceutiquement acceptables.
à l'exception du composé pour lequel R₂ et R₃ représentent des radicaux phényle, R₁ représente un radical -N(R₅)-Y-R₆, Y est SO₂, R₅ représente un radical méthyle et R₆ représente un radical phényle.

9 - Procédé de préparation des composés de formule (I) selon la revendication 5 pour lesquels R₁ représente un radical -NHCOR₄ **caractérisé en ce que** l'on fait réagir un acide R₄COOH pour lequel R₄ a les mêmes significations que dans la revendication 5 avec un dérivé de formule : 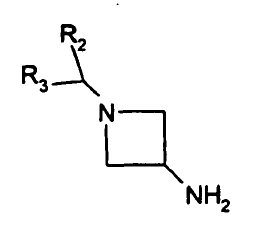 dans laquelle R₂ et R₃ ont les mêmes significations que dans la revendication 5, isole le prosuit et le transforme éventuellement en sel pharmaceutiquement acceptable.

10 - Procédé de préparation des composés de formule (I) selon la revendication 5 pour lesquels R₁ représente un radical -N(R₅)-Y-R₆ **caractérisé en ce que** l'on fait réagir un dérivé de formule : 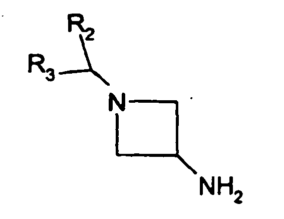 dans laquelle R₂ et R₃ ont les mêmes significations que dans la revendication 5 avec un dérivé Hal-Y-R₆ pour lequel Y et R₆ ont les mêmes significations que dans la revendication 5 et Hal représente un atome d'halogène, suivi éventuellement d'un dérivé Hal-alk, Hal représente un atome d'halogène et alk représente un radical alkyle (1-6C en chaîne droite ou ramifiée) pour obtenir les composés pour lesquels R₅ est alkyle, isole le prosuit et le transforme éventuellement en sel pharmaceutiquement acceptable.

11 - Procédé de préparation des composés de formule (I) selon la revendication 5 **caractérisé en ce que** l'on fait réagir un dérivé R₂-CHBr-R₃ pour lequel R₂ et R₃ ont les mêmes significations que dans la revendication 5, avec un dérivé de formule : 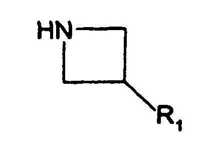 dans laquelle R₁ a les mêmes significations que dans la revendication 5, isole le prosuit et le transforme éventuellement en sel pharmaceutiquement acceptable.

12 - Procédé de préparation des composés de formule (I) selon la revendication 5 pour lesquels R, représente un radical -N(R₅)-Y-R₆ dans lequel R₆ est un radical phényle substitué par hydroxy **caractérisé en ce que** l'on hydrolyse un composé de formule (I) correspondant pour lequel R₁ représente un radical -N(R₅)-Y-R₆ dans lequel R₆ est un radical phényle substitué par alcoxy, isole le prosuit et le transforme éventuellement en sel pharmaceutiquement acceptable.

13 - Procédé de préparation des composés de formule (I) selon la revendication 5 pour lesquels R₁ représente un radical -N(R₅)-Y-R₆ dans lequel R₆ est un radical phényle substitué par hydroxyalkyle(1C) **caractérisé en ce que** l'on fait réagir l'hydrure de diisobutylaluminium sur un composé de formule (I) correspondant pour lequel R₁ représente un radical -N(R₅)-Y-R₆ dans lequel R₆ est un radical phényle substitué par alcoxycarbonyle, isole le prosuit et le transforme éventuellement en sel pharmaceutiquement acceptable.

14 - Procédé de préparation des composés de formule (I) selon la revendication 5 pour lesquels R₁ représente un radical -N(R₅)-Y-R₆ dans lequel R₆ est un radical phényle substitué par pyrrolidinyl-1 **caractérisé en ce que** l'on fait réagit de la pyrrolidine avec un composé de formule (I) correspondant pour lequel R₁ représente un radical -N(R₅)-Y-R₆ dans lequel R₆ est un radical phényle substitué par fluor, isole le prosuit et le transforme éventuellement en sel pharmaceutiquement acceptable.

## Revendications

1. Composition pharmaceutique contenant en tant qu'ingrédient actif un composé de formule : 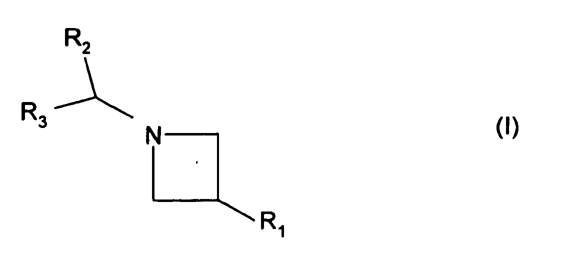 dans laquelle
R₁ représente un radical -NHCOR₄ ou -N(R₅)-Y-R₆,
Y est CO ou SO₂,
R₂ et R₃, identiques ou différents, représentent soit un aromatique choisi parmi phényle, naphtyle et indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, -COOalk, -CONR₇R₈, -CO-NH-NR₉R₁₀, alkylsulfanyle, alkylsulfinyle, alkylsulfonyle, alkylsulfanylalkyle, alkylsulfinylalkyle, alkylsulfonylalkyle, hydroxyalkyle, ou -alk-NR₇R₈; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, pyrimidinyle, furyle,
imidazolyle, isochromannyle, isoquinolyle, pyrrolyle, pyridyle, quinolyle, 1,2,3,4-tétrahydroisoquinolyle, thiazolyle et thiényle, ces hétéroaromatiques pouvant être non substitués ou substitués par un halogène, alkyle, alcoxy, hydroxy, trifluorométhyle, trifluorométhoxy, cyano, -COOH, -COOalk, -CO-NH-NR₉R₁₀, -CONR₇R₈, -alk-NR₉R₁₀, alkylsulfanyle, alkylsulfinyle, alkylsulfonyle, alkylsulfanylalkyle, alkylsulfinylalkyle, alkylsulfonylalkyle ou hydroxyalkyle,
R₄ représente un radical -alk-SO₂-R₁₁, -alk-SO₂-CH=CH-R₁₁, Het substitué par -SO₂-R₁₁ ou phényle substitué par -SO₂-R₁₁ ou -alk-SO₂-R₁₁,
R₅ représente un atome d'hydrogène ou un radical alkyle,
R₆ représente un radical phénylalkyle, Het ou Ar,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents représentent un atome d'hydrogène ou un radical alkyle, -COOalk, cycloalkyle, alkylcycloalkyle, -alk-O-alk ou hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, -CS-NHalk, oxo, hydroxyalkyle, -alk-O-alk ou -CO-NH₂,
R₁₁ représente un radical alkyle, Ar ou Het,
Ar représente un radical phényle, naphtyle ou indènyle, ces radicaux étant éventuellement substitués par un ou plusieurs halogène, alkyle, alcoxy, cyano, -CO-alk, -COOH, -COOalk, -CONR₁₂R₁₃, -CO-NH-NR₁₄R₁₅, alkylsulfanyle, alkylsulfinyle, alkylsulfonyle, -alk-NR₁₄R₁₅, -NR₁₄R₁₅, alkylthioalkyle, formyle, hydroxy, hydroxyalkyle, Het, -O-alk-NH-cycloalkyle, OCF₃, CF₃, -NH-CO-alk, -SO₂NH₂, -NH-COCH₃, -NH-COOalk, Het ou bien sur 2 atomes de carbone adjacents par un dioxyméthylène,
Het représente un hétérocycle mono ou bicyclique insaturé ou saturé, ayant 3 à 10 chaînons et contenant un ou plusieurs hétéroatomes choisis parmi oxygène, soufre et azote éventuellement substitué par un ou plusieurs alkyle, alcoxy, vinyle, halogène, alcoxycarbonyle, oxo, hydroxy, OCF₃ ou CF₃, les hétérocycles azotés étant éventuellement sous leur forme N-oxydée,
R₁₂ et R₁₃, identiques ou différents, représentent un atome d'hydrogène ou un radical alkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₁₄ et R₁₅, identiques ou différents, représentent un atome d'hydrogène ou un radical alkyle, -COOalk, cycloalkyle, alkylcycloalkyle, -alk-O-alk, hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, hydroxyalkyle, -alk-O-alk, -CO-NH₂,
alk représente un radical alkyle ou alkylène,
les radicaux et portions alkyle et alkylène et les radicaux et portions alcoxy sont en chaîne droite ou ramifiée et contiennent 1 à 6 atomes de carbone et les radicaux cycloalkyle contiennent 3 à 10 atomes de carbone,
un isomère optique d'un tel composé ou un de ses sels pharmaceutiquement acceptables.

2. Composition selon la revendication 1 pour laquelle dans le composé de formule (I) Het est choisi parmi benzimidazole, benzoxazole, benzothiazole, benzothiophène, cinnoline, thiophène, quinazoline, quinoxaline, quinoline, pyrazole, pyrrole, pyridine, imidazole, indole, isoquinoline, pyrimidine, thiazole, thiadiazole, pipéridine, pipérazine, pyrrolidine, triazole, furane, tétrahydroisoquinoline, tétrahydroquinoline, ces hétérocycles étant éventuellement substitués par un ou plusieurs alkyle, alcoxy, vinyle, halogène, alcoxycarbonyle, oxo, hydroxy, OCF₃ ou CF₃.

3. Composition pharmaceutique contenant en tant que principe actif au moins un composé de formule (I) selon la revendication 1 dans laquelle
R₁ représente un radical -N(R₅)-Y-R₆,
Y est SO₂,
R₂ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidinyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyle ou hydroxyalkyle,
R₃ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidinyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyleou hydroxyalkyle,
R₅ représente un atome d'hydrogène ou alkyle,
R₆ représente un radical naphtyle, phénylalkyle, Het ou phényle éventuellement substitué par un ou plusieurs halogène, alkyle, alcoxy, cyano, -CO-alk, COOalk, -CONR₁₂R₁₃, -alk-NR₁₄R₁₅, -NR₁₄R₁₅, hydroxy, hydroxyalkyle, Het, OCF₃, CF₃, -NH-CO-alk, -SO₂NH₂, -NH-COOalk, ou bien sur 2 atomes de carbone adjacents par dioxyméthylène,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents représentent un atome d'hydrogène ou un radical alkyle, cycloalkyle, alkylcycloalkyle ou hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, oxo ou -CO-NH₂,
R₁₂ et R₁₃, identiques ou différents, représentent un atome d'hydrogène ou un radical alkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₁₄ et R₁₅, identiques ou différents, représentent un atome d'hydrogène ou un radical alkyle, cycloalkyle, alkylcycloalkyle ou hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, oxo, hydroxyalkyle ou -CO-NH₂,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, vinyle, halogène, alcoxycarbonyle, oxo, hydroxy, les hétérocycles azotés étant éventuellement sous leur forme N-oxydée et, de préférence, Het représente un hétérocycle choisi parmi les hétérocycles suivants: benzimidazole, benzoxazole, benzothiazole; benzothiophène, thiophène, quinazoline, quinoxaline, quinoline, pyrrole, pyridine, imidazole, indole, isoquinoline, pyrimidine, thiazole, thiadiazole, furane, tétrahydroisoquinoline et tétrahydroquinoline, ces hétérocycles étant éventuellement substitués par un ou plusieurs alkyle, alcoxy, vinyle, halogène, oxo, hydroxy, OCF₃ ou CF₃,
un isomère optique d'un tel composé ou un de ses sels pharmaceutiquement acceptables.

4. Composition pharmaceutique contenant en tant que principe actif au moins un composé de formule (I) selon la revendication 1 dans laquelle
R₁ représente un radical -N(R₅)-Y-R₆,
Y est SO₂,
R₂ représente soit un phényle non substitué ou substitué par un ou plusieurs halogène, alkyle, alcoxy, trifluorométhyle, trifluorométhoxy ou hydroxyalkyle; soit un hétéroaromatique choisi parmi les cycles pyridyle et pyrimidyle, ces hétéroaromatiques pouvant être non substitués ou substitués par un halogène, alkyle, alcoxy, hydroxy, trifluorométhyle ou trifluorométhoxy,
R₃ représente soit un phényle non substitué ou substitué par un ou plusieurs halogène, alkyle, alcoxy, trifluorométhyle, trifluorométhoxy, hydroxyalkyle; soit un hétéroaromatique choisi parmi les cycles pyridyle et pyrimidyle, ces hétéroaromatiques pouvant être non substitués ou substitués par un halogène, alkyle, alcoxy, hydroxy, trifluorométhyle ou trifluorométhoxy,
R₅ représente un atome d'hydrogène ou alkyle,
R₆ représente un radical naphtyle, phénylalkyle, Het ou phényle éventuellement substitué par un ou plusieurs halogène, alkyle, alcoxy, -NR₁₄R₁₅, hydroxy, hydroxyalkyle, OCF₃, CF₃ ou -SO₂NH₂, ou bien sur 2 atomes de carbone adjacents par dioxyméthylène
R₁₄ et R₁₅, identiques ou différents représentent un atome d'hydrogène ou un radical alkyle, cycloalkyle, alkylcycloalkyle ou hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, oxo, hydroxyalkyle ou -CO-NH₂,
Het représente un hétérocycle mono où bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, vinyle, halogène, alcoxycarbonyle, oxo, hydroxy, les hétérocycles azotés étant éventuellement sous leur forme N-oxydée et, de préférence, Het représente un hétérocycle choisi parmi les hétérocycles suivants : benzimidazole, benzoxazole, benzothiazole, benzothiophène, thiophène, quinoline, pyrrole, pyridine, pyrimidine, thiazole, thiadiazole, furane, tétrahydroisoquinoline, tétrahydroquinoline, ces hétérocycles étant éventuellement substitués par un ou plusieurs alkyle, alcoxy, vinyle, halogène, oxo, hydroxy, OCF₃ ou CF₃,
un isomère optique d'un tel composé ou un de ses sels pharmaceutiquement acceptables.

5. Composés de formule : 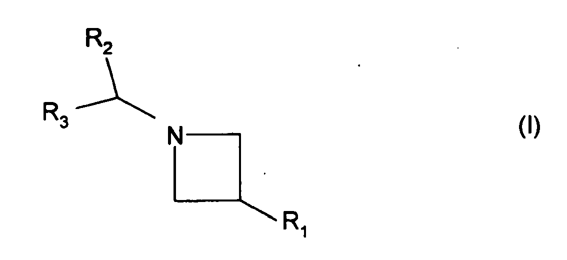 dans laquelle
R₁ représente un radical -NHCOR₄ ou -N(R₅)-Y-R₆,
Y est CO ou SO₂,
R₂ et R₃, identiques ou différents, représentent soit un aromatique choisi parmi phényle, naphtyle et indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, -COOalk, -CONR₇R₈, -CO-NH-NR₉R₁₀, alkylsulfanyle, alkylsulfinyle, alkylsulfonyle, alkylsulfanylalkyle, alkylsulfinylalkyle, alkylsulfonylalkyle, hydroxyalkyle, ou -alk-NR₇R₈; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, pyrimidinyle, furyle,
imidazolyle, isochromannyle, isoquinolyle, pyrrolyle, pyridyle, quinolyle, 1,2,3,4-tétrahydroisoquinolyle, thiazolyle et thiényle, ces hétéroaromatiques pouvant être non substitués ou substitués par un halogène, alkyle, alcoxy, hydroxy, trifluorométhyle, trifluorométhoxy, cyano, -COOH, -COOalk, -CO-NH-NR₉R₁₀, -CONR₇R₈, -alk-NR₉R₁₀, alkylsulfanyle, alkylsulfinyle, alkylsulfonyle, alkylsulfanylalkyle, alkylsulfinylalkyle, alkylsulfonylalkyle ou hydroxyalkyle,
R₄ représente un radical -alk-SO₂-R₁₁, -alk-SO₂-CH=CH-R₁₁, Het substitué par -SO₂-R₁₁ ou phényle substitué par -SO₂-R₁₁ ou -alk-SO₂-R₁₁,
R₅ représente un atome d'hydrogène ou un radical alkyle,
R₆ représente un radical phénylalkyle, Het ou Ar,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents représentent un atome d'hydrogène ou un radical alkyle, -COOalk, cycloalkyle, alkylcycloalkyle, -alk-O-alk ou hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, -CS-NHalk, oxo, hydroxyalkyle, -alk-O-alk ou -CO-NH₂,
R₁₁ représente un radical alkyle, Ar ou Het,
Ar représente un radical phényle, naphtyle ou indènyle, ces radicaux étant éventuellement substitués par un ou plusieurs halogène, alkyle, alcoxy, cyano, -CO-alk, -COOH, -COOalk, -CONR₁₂R₁₃, -CO-NH-NR₁₄R₁₅, alkylsulfanyle, alkylsulfinyle, alkylsulfonyle, -alk-NR₁₄R₁₅, -NR₁₄R₁₅, alkylthioalkyle, formyle, hydroxy, hydroxyalkyle, Het, -O-alk-NH-cycloalkyle, OCF₃, CF₃, -NH-CO-alk, -SO₂NH₂, -NH-COCH₃, -NH-COOalk, Het ou bien sur 2 atomes de carbone adjacents par un dioxyméthylène,
Het représente un hétérocycle mono ou bicyclique insaturé ou saturé, ayant 3 à 10 chaînons et contenant un ou plusieurs hétéroatomes choisis parmi oxygène, soufre et azote éventuellement substitué par un ou plusieurs alkyle, alcoxy, vinyle, halogène, alcoxycarbonyle, oxo, hydroxy, OCF₃ ou CF₃, les hétérocycles azotés étant éventuellement sous leur forme N-oxydée,
R₁₂ et R₁₃, identiques ou différents, représentent un atome d'hydrogène ou un radical alkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₁₄ et R₁₅, identiques ou différents, représentent un atome d'hydrogène ou un radical alkyle, -COOalk, cycloalkyle, alkylcycloalkyle, -alk-O-alk, hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, hydroxyalkyle, -alk-O-alk, -CO-NH₂,
alk représente un radical alkyle ou alkylène,
les radicaux et portions alkyle et alkylène et les radicaux et portions alcoxy sont en chaîne droite ou ramifiée et contiennent 1 à 6 atomes de carbone et les radicaux cycloalkyle contiennent 3 à 10 atomes de carbone,
leurs isomères optiques et leurs sels pharmaceutiquement acceptables,
à l'exception du composé pour lequel pour lequel R₂ et R₃ représentent des radicaux phényle, R₁ représente un radical -N(R₅)-Y-R₆, Y est SO₂, R₅ représente un radical méthyle et R₆ représente un radical phényle.

6. Composés de formule (I) selon la revendication 5 pour lequel Het est choisi parmi benzimidazole, benzoxazole, benzothiazole, benzothiophène, cinnoline, thiophène, quinazoline, quinoxaline, quinoline, pyrazole, pyrrole, pyridine, imidazole, indole, isoquinoline, pyrimidine, thiazole, thiadiazole, pipéridine, pipérazine, pyrrolidine, triazole, furane, tétrahydroisoquinoline, tétrahydroquinoline, ces hétérocycles étant éventuellement substitués par un ou plusieurs alkyle, alcoxy, vinyle, halogène, alcoxycarbonyle, oxo, hydroxy, OCF₃ ou CF₃ pour lequel R₂ et R₃ représentent des radicaux phényle, R₁ représente un radical -N(R₅)-Y-R₆, Y est SO₂, R₅ représente un radical méthyle et R₆ représente un radical phényle.

7. Composés de formule (I) selon la revendication 5 dans laquelle
R₁ représente un radical -N(R₅)-Y-R₆,
Y est SO₂,
R₂ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidinyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyle ou hydroxyalkyle,
R₃ représente soit un phényle non substitué ou substitué par un ou plusieurs halogène, alkyle, alcoxy, trifluorométhyle, trifluorométhoxy, cyano, -CONR₇R₈, hydroxyalkyle ou -alk-NR₇R₈; soit un hétéroaromatique choisi parmi les cycles pyridyle, pyrimidinyle, thiazolyle et thiényle, ces hétéroaromatiques pouvant être non substitués ou substitués par un halogène, alkyle, alcoxy, hydroxy, trifluorométhyle, trifluorométhoxy, -CONR₇R₈, -alk-NR₉R₁₀, alkylsulfanyle, alkylsulfinyle, alkylsulfonyleou hydroxyalkyle,
R₅ représente un atome d'hydrogène ou alkyle,
R₆ représente un radical naphtyle, phénylalkyle, Het ou phényle éventuellement substitué par un ou plusieurs halogène, alkyle, alcoxy, cyano, -CO-alk, COOalk, -CONR₁₂R₁₃, -alk-NR₁₄R₁₅, -NR₁₄R₁₅, hydroxy, hydroxyalkyle, Het, OCF₃, CF₃, -NH-CO-alk, -SO₂NH₂, -NH-COOalk, ou bien sur 2 atomes de carbone adjacents par dioxyméthylène,
R₇ et R₈, identiques ou différents, représentent un atome d'hydrogène ou un radical alkyle ou bien R₇ et R₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₉ et R₁₀, identiques ou différents représentent un atome d'hydrogène ou un radical alkyle, cycloalkyle, alkylcycloalkyle ou hydroxyalkyle ou bien R₉ et R₁₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, oxo ou -CO-NH₂,
R₁₂ et R₁₃, identiques ou différents, représentent un atome d'hydrogène ou un radical alkyle ou bien R₁₂ et R₁₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₁₄ et R₁₅, identiques ou différents, représentent un atome d'hydrogène ou un radical alkyle, cycloalkyle, alkylcycloalkyle ou hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, oxo, hydroxyalkyle ou -CO-NH₂,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, vinyle, halogène, alcoxycarbonyle, oxo, hydroxy, les hétérocycles azotés étant éventuellement sous leur forme N-oxydée et, de préférence, Het représente un hétérocycle choisi parmi les hétérocycles suivants : benzimidazole, benzoxazole, benzothiazole, benzothiophène, thiophène, quinazoline, quinoxaline, quinoline, pyrrole, pyridine, imidazole, indole, isoquinoline, pyrimidine, thiazole, thiadiazole, furane, tétrahydroisoquinoline et tétrahydroquinoline, ces hétérocycles étant éventuellement substitués par un ou plusieurs alkyle, alcoxy, vinyle, halogène, oxo, hydroxy, OCF₃ ou CF₃,
leurs isomères optiques et leurs sels pharmaceutiquement acceptables,
à l'exception du composé pour lequel R₂ et R₃ représentent des radicaux phényle, R₁ représente un radical -N(R₅)-Y-R₆, Y est SO₂, R₅ représente un radical méthyle et R₆ représente un radical phényle.

8. Composés de formule (I) selon la revendication 5 dans laquelle
R₁ représente un radical -N(R₅)-Y-R₆,
Y est SO₂,
R₂ représente soit un phényle non substitué ou substitué par un ou plusieurs halogène, alkyle, alcoxy, trifluorométhyle, trifluorométhoxy ou hydroxyalkyle;
soit un hétéroaromatique choisi parmi les cycles pyridyle et pyrimidyle, ces hétéroaromatiques pouvant être non substitués ou substitués par un halogène, alkyle, alcoxy, hydroxy, trifluorométhyle ou trifluorométhoxy,
R₃ représente soit un phényle non substitué ou substitué par un ou plusieurs halogène, alkyle, alcoxy, trifluorométhyle, trifluorométhoxy, hydroxyalkyle; soit un hétéroaromatique choisi parmi les cycles pyridyle et pyrimidyle, ces hétéroaromatiques pouvant être non substitués ou substitués par un halogène, alkyle, alcoxy, hydroxy, trifluorométhyle ou trifluorométhoxy,
R₅ représente un atome d'hydrogène ou alkyle,
R₆ représente un radical naphtyle, phénylalkyle, Het ou phényle éventuellement substitué par un ou plusieurs halogène, alkyle, alcoxy, -NR₁₄R₁₅, hydroxy, hydroxyalkyle, OCF₃, CF₃ ou -SO₂NH₂, ou bien sur 2 atomes de carbone adjacents par dioxyméthylène
R₁₄ et R₁₅, identiques ou différents représentent un atome d'hydrogène ou un radical alkyle, cycloalkyle, alkylcycloalkyle ou hydroxyalkyle ou bien R₁₄ et R₁₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, oxo, hydroxyalkyle ou -CO-NH₂,
Het représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, vinyle, halogène, alcoxycarbonyle, oxo, hydroxy, les hétérocycles azotés étant éventuellement sous leur forme N-oxydée et, de préférence, Het représente un hétérocycle choisi parmi les hétérocycles suivants : benzimidazole, benzoxazole, benzothiazole, benzothiophène, thiophène, quinoline, pyrrole, pyridine, pyrimidine, thiazole, thiadiazole, furane, tétrahydroisoquinoline, tétrahydroquinoline, ces hétérocycles étant éventuellement substitués par un ou plusieurs alkyle, alcoxy, vinyle, halogène, oxo, hydroxy, OCF₃ ou CF₃,
leurs isomères optiques et leurs sels pharmaceutiquement acceptables.
à l'exception du composé pour lequel R₂ et R₃ représentent des radicaux phényle, R₁ représente un radical -N(R₅)-Y-R₆, Y est SO₂, R₅ représente un radical méthyle et R₆ représente un radical phényle.

9. Composé de formule (I) selon l'une des revendications 1 à 8 **caractérisé en ce qu'**il est choisi parmi :
• N-{1-[bis(4-chlorophényl)méthyl]azétidin-3-yl}-thièn-2-yl-sulfonamide ;
• N-{1-[bis(4-chlorophényl)méthyl]azétidin-3-yl}-4-méthoxyphénylsulfonamide ;
• N-[4-(N-{1-[bis(4-chlorophényl)méthyl]azétidin-3-yl}sulfamoyl)phényl]acétamide ;
• N-{1-[bis(4-chlorophényl)méthyl]azétidin-3-yl}-4-méthylphénylsulfonamide ;
• N-{1-[bis(4-chlorophényl)méthyl]azétidin-3-yl}-3,4-diméthoxyphénylsulfonamide ;
• N-{1-[bis(4-chlorophényl)méthyl]azétidin-3-yl}-3-fluorophénylsulfonamide ;
• N-{1-[bis(4-chlorophényl)méthyl]azétidin-3-yl}-3,4-dichlorophénylsulfonamide ;
• N-{1-[bis(4-chlorophényl)méthyl]azétidin-3-yl}-3-cyanophénylsulfonamide ;
• N-{1-[bis(4-chlorophényl)méthyl]azétidin-3-yl}-2,5-diméthoxyphénylsulfonamide ;
• N-{1-[bis(4-chlorophényl)méthyl]azétidin-3-yl}-3-trifluorométhylphénylsulfonamide ;
• N-{1-[bis(4-chlorophényl)méthyl]azétidin-3-yl}-napht-2-yl-sulfonamide ;
• N-{1-[bis(4-chlorophényl)méthyl]azétidin-3-yl}napht-1-yl-sulfonamide ;
• N-{1-[bis(4-chlorophényl)méthyl]azétidin-3-yl]}-3,4-difluorophénylsulfonamide ;
• N-{1-[bis(4-chlorophényl)méthyl]azétidin-3-yl}-1-méthyl-1-H-imidazol-4-yl-sulfonamide ;
• N-[4-(N-{1-[bis(4-chlorophényl)méthyl]azétidin-3-yl}sulfamoyl)-2-chlorophényl]acétamide ;
• N-{1-[bis(4-chlorophényl)méthyl]azétidin-3-yl}pyrid-3-yl-sulfonamide ;
• N-{1-[bis(4-chlorophényl)méthyl]azétidin-3-yl}-4-fluorophénylsulfonamide ;
• N-{1-[bis(4-chlorophényl)méthyl]azétidin-3-yl}quinol-8-ylsulfonamide ;
• N-{1-[bis(4-chlorophényl)méthyl]azétidin-3-yl}phénylsulfonamide ;
• N-{1-[bis(4-chlorophényl)méthyl]azétidin-3-yl}-(phénylméthyl)sulfonamide ;
• N-{1-[bis(4-chlorophényl)méthyl]azétidin-3-yl}-3,5-difluorophénylsulfonamide ;
• N-{1-[bis(4-chlorophényl)méthyl]azétidin-3-yl}pyrid-2-ylsulfonamide ;
• N-{1-[bis(4-chlorophényl)méthyl]azétidin-3-yl}-(3-fluoro-5-pyrrolidin-1-yl-phényl)sulfonamide ;
• N-{1-[bis(4-chlorophényl)méthyl]azétidin-3-yl}-N-méthyl-4-fluorophénylsulfonamide ;
• N-{1-[bis(4-chlorophényl)méthyl]azétidin-3-yl}-N-méthyl-quinol-8-ylsulfonamide ;
• N-{1-[bis(4-chlorophényl)méthyl]azétidin-3-yl}-N-méthyl-phénylsulfonamide ;
• N-{1-[bis(4-chlorophényl)méthyl]azétidin-3-yl}-N-méthyl-(phénylméthyl)sulfonamide ;
• N-{1-[bis(4-chlorophényl)méthyl]azétidin-3-yl}-3-sulfamoylphénylsulfonamide ;
• 2-benzènesulfonyl-N-{1-[bis-(4-chlorophényl)-méthyl]-azétidin-3-yl}-acétamide ;
• N-{1-[bis-(4-chlorophényl)-méthyl]-azétidin-3-yl}-2-(toluène-4-sulfonyl)-acétamide ;
• N-{1-[bis-(4-chlorophényl)-méthyl]-azétidin-3-yl}-2-(toluène-4-sulfonyl)-acétamide;
• N-{1-[bis-(4-chlorophényl)-méthyl]-azétidin-3-yl}-3-(2-phényl-éthylènesulfonyl)-propionamide ;
• N-{1-[bis-(4-chlorophényl)-méthyl]-azétidin-3-yl}-4-méthanesulfonyl-benzamide ;
• N-{1-[bis-(4-chlorophényl)-méthyl]-azétidin-3-yl}-4-méthanesulfonyl-benzamide ;
• (5-méthylsulfonyl-thiophène-2-carboxy)-{1-[bis-(4-chlorophényl)-méthyl]-azétidin-3-yl}-amide ;
• (5-méthylsulfonyl-3-méthyl-4-vinyl-thiophène-2-carboxy)-{1-[bis-(4-chlorophényl)-méthyl]-azétidin-3-yl}-amide ;
• N-{1-[bis-(4-chlorophényl)-méthyl]-azétidin-3-yl}-3-méthylsulfonylméthyl-benzamide ;
• (RS)-N-{1-[(4-chloro-phényl)-pyridin-3-yl-méthyl]-azétidin-3-yl}-3,5-difluoro-benzènesulfonamide ;
• (RS)-N-{1-[(4-chloro-phényl)-pyrimidin-5-yl-méthyl]-azétidin-3-yl}-3,5-difluoro-benzenesulfonamide.

10. Procédé de préparation des composés de formule (I) selon la revendication 5 pour lesquels R₁ représente un radical -NHCOR₄ **caractérisé en ce que** l'on fait réagir un acide R₄COOH pour lequel R₄ a les mêmes significations que dans la revendication 5 avec un dérivé de formule : 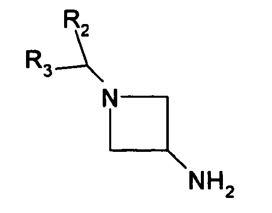 dans laquelle R₂ et R₃ ont les mêmes significations que dans la revendication 5, isole le prosuit et le transforme éventuellement en sel pharmaceutiquement acceptable.

11. Procédé de préparation des composés de formule (I) selon la revendication 5 pour lesquels R₁ représente un radical -N(R₅)-Y-R₆ **caractérisé en ce que** l'on fait réagir un dérivé de formule : 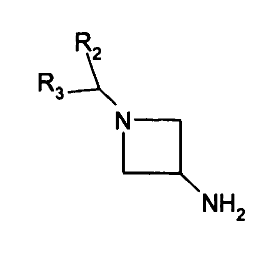 dans laquelle R₂ et R₃ ont les mêmes significations que dans la revendication 5 avec un dérivé Hal-Y-R₆ pour lequel Y et R₆ ont les mêmes significations que dans la revendication 5 et Hal représente un atome d'halogène, suivi éventuellement d'un dérivé Hal-alk, Hal représente un atome d'halogène et alk représente un radical alkyle (1-6C en chaîne droite ou ramifiée) pour obtenir les composés pour lesquels R₅ est alkyle, isole le prosuit et le transforme éventuellement en sel pharmaceutiquement acceptable.

12. Procédé de préparation des composés de formule (I) selon la revendication 5 **caractérisé en ce que** l'on fait réagir un dérivé R₂-CHBr-R₃ pour lequel R₂ et R₃ ont les mêmes significations que dans la revendication 5, avec un dérivé de formule : 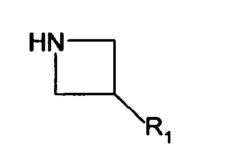 dans laquelle R₁ a les mêmes significations que dans la revendication 5, isole le prosuit et le transforme éventuellement en sel pharmaceutiquement acceptable.

13. Procédé de préparation des composés de formule (I) selon la revendication 5 pour lesquels R₁ représente un radical -N(R₅)-Y-R₆ dans lequel R₆ est un radical phényle substitué par hydroxy **caractérisé en ce que** l'on hydrolyse un composé de formule (I) correspondant pour lequel R₁ représente un radical -N(R₅)-Y-R₆ dans lequel R₆ est un radical phényle substitué par alcoxy, isole le prosuit et le transforme éventuellement en sel pharmaceutiquement acceptable.

14. Procédé de préparation des composés de formule (I) selon la revendication 5 pour lesquels R₁ représente un radical -N(R₅)-Y-R₆ dans lequel R₆ est un radical phényle substitué par hydroxyalkyle(1C) **caractérisé en ce que** l'on fait réagir l'hydrure de diisobutylaluminium sur un composé de formule (I) correspondant pour lequel R₁ représente un radical -N(R₅)-Y-R₆ dans lequel R₆ est un radical phényle substitué par alcoxycarbonyle, isole le prosuit et le transforme éventuellement en sel pharmaceutiquement acceptable.

15. Procédé de préparation des composés de formule (I) selon la revendication 5 pour lesquels R₁ représente un radical -N(R₅)-Y-R₆ dans lequel R₆ est un radical phényle substitué par pyrrolidinyl-1 **caractérisé en ce que** l'on fait réagit de la pyrrolidine avec un composé de formule (I) correspondant pour lequel R₁ représente un radical -N(R₅)-Y-R₆ dans lequel R₆ est un radical phényle substitué par fluor, isole le prosuit et le transforme éventuellement en sel pharmaceutiquement acceptable.

16. Médicament **caractérisé en ce qu'**il contient en tant que principe actif au moins un composé de formule (I) selon l'une des revendications 1 à 9.

## Claims

1. Pharmaceutical composition containing, as active ingredient, a compound of formula: 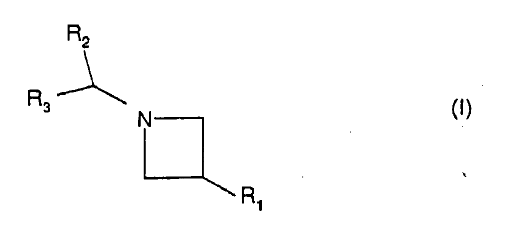 in which
R₁ represents a radical -NHCOR₄ or -N(R₅)-Y-R₆,
Y is CO or SO₂,
R₂ and R₃, which are identical or different, represent either an aromatic chosen from phenyl, naphthyl and indenyl, these aromatics being unsubstituted or substituted with one or more halogen atoms or alkyl, alkoxy, formyl, hydroxyl, trifluoromethyl, trifluoromethoxy, -CO-alk, cyano, -COOH, -COOalk, -CONR₇R₈, -CO-NH-NR₉R₁₀, alkylsulfanyl, alkylsulfinyl, alkylsulfonyl, alkylsulfanylalkyl, alkylsulfinylalkyl, alkylsulfonylalkyl, hydroxyalkyl or -alk-NR₇R₈ radicals;
or a heteroaromatic chosen from benzofuryl, benzothiazolyl, benzothienyl, benzoxazolyl, chromanyl, 2,3-dihydrobenzofuryl, 2,3-dihydrobenzothienyl, pyrimidinyl, furyl, imidazolyl, isochromanyl, isoquinolyl, pyrrolyl, pyridyl, quinolyl, 1,2,3,4-tetrahydroisoquinolyl, thiazolyl and thienyl rings, it being possible for these heteroaromatics to be unsubstituted or substituted with a halogen atom or an alkyl, alkoxy, hydroxyl, trifluoromethyl, trifluoromethoxy, cyano, -COOH, -COOalk, -CO-NH-NR₉R₁₀, -CONR₇R₈, -alk-NR₉R₁₀, alkylsulfanyl, alkylsulfinyl, alkylsulfonyl, alkylsulfanylalkyl, alkylsulfinylalkyl, alkylsulfonylalkyl or hydroxyalkyl radical,
R₄ represents a radical -alk-SO₂-R₁₁, -alk-SO₂-CH=CH-R₁₁, Het substituted with -SO₂-R₁₁ or phenyl substituted with -SO₂-R₁₁ or -alk-SO₂-R₁₁,
R₅ represents a hydrogen atom or an alkyl radical,
R₆ represents a phenylalkyl, Het or Ar radical,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₉ and R₁₀, which are identical or different, represent a hydrogen atom or an alkyl, -COOalk, cycloalkyl, alkylcycloalkyl, -alk-O-alk or hydroxyalkyl radical or alternatively R₉ and R₁₀ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, -CS-NHalk, oxo, hydroxyalkyl, -alk-O-alk or -CO-NH₂ radicals,
R₁₁ represents an alkyl, Ar or Het radical,
Ar represents a phenyl, naphthyl or indenyl radical, these radicals being optionally substituted with one or more halogen atoms or alkyl, alkoxy, cyano, -CO-alk, -COOH, -COOalk, -CONR₁₂R₁₃, -CO-NH-NR₁₄R₁₅, alkylsulfanyl, alkylsulfinyl, alkylsulfonyl, -alk-NR₁₄R₁₅, -NR₁₄R₁₅, alkylthioalkyl, formyl, hydroxyl, hydroxyalkyl, Het, -O-alk-NH-cycloalkyl, OCF₃, CF₃, -NH-CO-alk, -SO₂NH₂, -NH-COCH₃, -NH-COOalk or Het radicals or alternatively, on 2 adjacent carbon atoms, with a dioxymethylene,
Het represents a 3- to 10-membered unsaturated or saturated mono- or bicyclic heterocycle containing one or more heteroatoms chosen from oxygen, sulfur and nitrogen optionally substituted with one or more halogen atoms or alkyl, alkoxy, vinyl, alkoxycarbonyl, oxo, hydroxyl, OCF₃ or CF₃ radicals, the nitrogen-containing heterocycles being optionally in their N-oxidized form,
R₁₂ and R₁₃, which are identical or different, represent a hydrogen atom or an alkyl radical or alternatively R₁₂ and R₁₃ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₄ and R₁₅, which are identical or different, represent a hydrogen atom or an alkyl, -COOalk, cycloalkyl, alkylcycloalkyl, -alk-O-alk or hydroxyalkyl radical or alternatively R₁₄ and R₁₅ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, -CS-NHalk, oxo, hydroxyalkyl, -alk-O-alk or -CO-NH₂ radicals,
alk represents an alkyl or alkylene radical,
the alkyl and alkylene radicals and portions and the alkoxy radicals and portions are in the form of a straight or branched chain and contain 1 to 6 carbon atoms and the cycloalkyl radicals contain 3 to 10 carbon atoms,
an optical isomer of such a compound or one of its pharmaceutically acceptable salts.

2. Composition according to Claim 1 for which in the compound of formula (I) Het is chosen from benzimidazole, benzoxazole, benzothiazole, benzothiophene, cinnoline, thiophene, quinazoline, quinoxaline, quinoline, pyrazole, pyrrole, pyridine, imidazole, indole, isoquinoline, pyrimidine, thiazole, thiadiazole, piperidine, piperazine, pyrrolidine, triazole, furan, tetrahydroisoquinoline, tetrahydroquinoline, these heterocycles being optionally substituted with one or more halogen atoms or alkyl, alkoxy, vinyl, alkoxycarbonyl, oxo, hydroxyl, OCF₃ or CF₃ radicals.

3. Pharmaceutical composition containing, as active ingredient, at least one compound of formula (I) according to Claim 1 in which
R₁ represents a radical -N(R₅)-Y-R₆,
Y is SO₂,
R₂ represents either a phenyl which is unsubstituted or substituted with one or more halogen atoms or alkyl, alkoxy, trifluoromethyl, trifluoromethoxy, cyano, -CONR₇R₈, hydroxyalkyl or -alk-NR₇R₈ radicals; or a heteroaromatic chosen from the pyridyl, pyrimidinyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈, -alk-NR₉R₁₀, alkylsulfanyl, alkylsulfinyl, alkylsulfonyl or hydroxyalkyl radical,
R₃ represents either a phenyl which is unsubstituted or substituted with one or more halogen atoms or alkyl, alkoxy, trifluoromethyl, trifluoromethoxy, cyano, -CONR₇R₈, hydroxyalkyl or -alk-NR₇R₈ radicals; or a heteroaromatic chosen from the pyridyl, pyrimidinyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈, -alk-NR₉R₁₀, alkylsulfanyl, alkylsulfinyl, alkylsulfonyl or hydroxyalkyl radical,
R₅ represents a hydrogen atom or an alkyl radical,
R₆ represents a naphthyl, phenylalkyl, Het or phenyl radical optionally substituted with one or more halogen atoms or alkyl, alkoxy, cyano, -CO-alk, COOalk, -CONR₁₂R₁₃, -alk-NR₁₄R₁₅, -NR₁₄R₁₅, hydroxyl, hydroxyalkyl, Het, OCF₃, CF₃, -NH-CO-alk, -SO₂NH₂ or -NH-COOalk radicals, or alternatively, on 2 adjacent carbon atoms, with dioxymethylene,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₉ and R₁₀, which are identical or different, represent a hydrogen atom or an alkyl, cycloalkyl, alkylcycloalkyl or hydroxyalkyl radical or alternatively R₉ and R₁₀ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, oxo or -CO-NH₂ radicals,
R₁₂ and R₁₃, which are identical or different, represent a hydrogen atom or an alkyl radical or alternatively R₁₂ and R₁₃ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₄ and R₁₅, which are identical or different, represent a hydrogen atom or an alkyl, cycloalkyl, alkylcycloalkyl or hydroxyalkyl radical or alternatively R₁₄ and R₁₅ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, oxo, hydroxyalkyl or -CO-NH₂ radicals,
Het represents a 3- to 10-membered unsaturated or saturated mono- or bicyclic heterocycle containing one or more heteroatoms chosen from oxygen, sulfur and nitrogen optionally substituted with one or more halogen atoms or alkyl, alkoxy, vinyl, alkoxycarbonyl, oxo or hydroxyl radicals, the nitrogen-containing heterocycles being optionally in their N-oxidized form and, preferably, Het represents a heterocycle chosen from the following heterocycles: benzimidazole, benzoxazole, benzothiazole, benzothiophene, thiophene, quinazoline, quinoxaline, quinoline, pyrrole, pyridine, imidazole, indole, isoquinoline, pyrimidine, thiazole, thiadiazole, furan, tetrahydroisoquinoline and tetrahydroquinoline, these heterocycles being optionally substituted with one or more halogen atoms or alkyl, alkoxy, vinyl, oxo, hydroxyl, OCF₃ or CF₃ radicals,
an optical isomer of such a compound or one of its pharmaceutically acceptable salts.

4. Pharmaceutical composition containing, as active ingredient, at least one compound of formula (I) according to Claim 1 in which
R₁ represents a radical -N(R₅)-Y-R₆,
Y is SO₂,
R₂ represents either a phenyl which is unsubstituted or substituted with one or more halogen atoms or alkyl, alkoxy, trifluoromethyl, trifluoromethoxy or hydroxyalkyl radicals; or a heteroaromatic chosen from pyridyl and pyrimidyl rings, it being possible for these heteroaromatics to be unsubstituted or substituted with a halogen atom or an alkyl, alkoxy, hydroxyl, trifluoromethyl or trifluoromethoxy radical, R₃ represents either a phenyl which is unsubstituted or substituted with one or more halogen atoms or alkyl, alkoxy, trifluoromethyl, trifluoromethoxy or hydroxyalkyl radicals; or a heteroaromatic chosen from pyridyl and pyrimidyl rings, it being possible for these heteroaromatics to be unsubstituted or substituted with a halogen atom or an alkyl, alkoxy, hydroxyl, trifluoromethyl or trifluoromethoxy radical, R₅ represents a hydrogen atom or an alkyl radical,
R₆ represents a naphthyl, phenylalkyl, Het or phenyl radical optionally substituted with one or more halogen atoms or alkyl, alkoxy, -NR₁₄R₁₅, hydroxyl, hydroxyalkyl, OCF₃, CF₃ or -SO₂NH₂ radicals, or alternatively, on 2 adjacent carbon atoms, with dioxymethylene,
R₁₄ and R₁₅, which are identical or different, represent a hydrogen atom or an alkyl, cycloalkyl, alkylcycloalkyl or hydroxyalkyl radical or alternatively R₁₄ and R₁₅ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, oxo, hydroxyalkyl or -CO-NH₂ radicals,
Het represents a 3- to 10-membered unsaturated or saturated mono- or bicyclic heterocycle containing one or more heteroatoms chosen from oxygen, sulfur and nitrogen optionally substituted with one or more halogen atoms or alkyl, alkoxy, vinyl, alkoxycarbonyl, oxo or hydroxyl radicals, the nitrogen-containing heterocycles being optionally in their N-oxidized form and, preferably, Het represents a heterocycle chosen from the following heterocycles: benzimidazole, benzoxazole, benzothiazole, benzothiophene, thiophene, quinoline, pyrrole, pyridine, pyrimidine, thiazole, thiadiazole, furan, tetrahydroisoquinoline and tetrahydroquinoline, these heterocycles being optionally substituted with one or more halogen atoms or alkyl, alkoxy, vinyl, oxo, hydroxyl, OCF₃ or CF₃ radicals,
an optical isomer of such a compound or one of its pharmaceutically acceptable salts.

5. Compounds of formula: 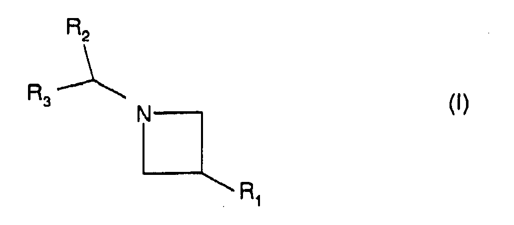 in which
R₁ represents a radical -NHCOR₄ or -N(R₅)-Y-R₆,
Y is CO or SO₂,
R₂ and R₃, which are identical or different, represent either an aromatic chosen from phenyl, naphthyl and indenyl, these aromatics being unsubstituted or substituted with one or more halogen atoms or alkyl, alkoxy, formyl, hydroxyl, trifluoromethyl, trifluoromethoxy, -CO-alk, cyano, -COOH, -COOalk, -CONR₇R₈, -CO-NH-NR₉R₁₀, alkylsulfanyl, alkylsulfinyl, alkylsulfonyl, alkylsulfanylalkyl, alkylsulfinylalkyl, alkylsulfonylalkyl, hydroxyalkyl or -alk-NR₇R₈ radicals;
or a heteroaromatic chosen from benzofuryl, benzothiazolyl, benzothienyl, benzoxazolyl, chromanyl, 2,3-dihydrobenzofuryl, 2,3-dihydrobenzothienyl, pyrimidinyl, furyl, imidazolyl, isochromanyl, isoquinolyl, pyrrolyl, pyridyl, quinolyl, 1,2,3,4-tetrahydro-isoquinolyl, thiazolyl and thienyl rings, it being possible for these heteroaromatics to be unsubstituted or substituted with a halogen atom or an alkyl, alkoxy, hydroxyl, trifluoromethyl, trifluoromethoxy, cyano, -COOH, -COOalk, -CO-NH-NR₉R₁₀, -CONR₇R₈, -alk-NR₉R₁₀, alkylsulfanyl, alkylsulfinyl, alkylsulfonyl, alkylsulfanylalkyl, alkylsulfinylalkyl, alkylsulfonylalkyl or hydroxyalkyl radical,
R₄ represents a radical -alk-SO₂-R₁₁, -alk-SO₂-CH=CH-R₁₁, Het substituted with -SO₂-R₁₁ or phenyl substituted with -SO₂-R₁₁ or -alk-SO₂-R₁₁,
R₅ represents a hydrogen atom or an alkyl radical,
R₆ represents a phenylalkyl, Het or Ar radical,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₉ and R₁₀, which are identical or different, represent a hydrogen atom or an alkyl, -COOalk, cycloalkyl, alkylcycloalkyl, -alk-O-alk or hydroxyalkyl radical or alternatively R₉ and R₁₀ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, -CS-NHalk, oxo, hydroxyalkyl, -alk-O-alk or -CO-NH₂ radicals,
R₁₁ represents an alkyl, Ar or Het radical,
Ar represents a phenyl, naphthyl or indenyl radical, these radicals being optionally substituted with one or more halogen atoms or alkyl, alkoxy, cyano, -CO-alk, -COOH, -COOalk, -CONR₁₂R₁₃, -CO-NH-NR₁₄R₁₅, alkylsulfanyl, alkylsulfinyl, alkylsulfonyl, -alk-NR₁₄R₁₅, -NR₁₄R₁₅, alkylthioalkyl, formyl, hydroxyl, hydroxyalkyl, Het, -O-alk-NH-cycloalkyl, OCF₃, CF₃, -NH-CO-alk, -SO₂NH₂, -NH-COCH₃, -NH-COOalk or Het radicals, or alternatively, on 2 adjacent carbon atoms, with a dioxymethylene,
Het represents a 3- to 10-membered unsaturated or saturated mono- or bicyclic heterocycle containing one or more heteroatoms chosen from oxygen, sulfur and nitrogen optionally substituted with one or more halogen atoms or alkyl, alkoxy, vinyl, alkoxycarbonyl, oxo, hydroxyl, OCF₃ or CF₃ radicals, the nitrogen-containing heterocycles being optionally in their N-oxidized form,
R₁₂ and R₁₃, which are identical or different, represent a hydrogen atom or an alkyl radical or alternatively R₁₂
and R₁₃ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₄ and R₁₅, which are identical or different, represent a hydrogen atom or an alkyl, -COOalk, cycloalkyl, alkylcycloalkyl, -alk-O-alk or hydroxyalkyl radical or alternatively R₁₄ and R₁₅ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, -COalk, -COOalk, -CO-NHalk, -CS-NHalk, oxo, hydroxyalkyl, -alk-O-alk or -CO-NH₂ radicals,
alk represents an alkyl or alkylene radical,
the alkyl and alkylene radicals and portions and the alkoxy radicals and portions are in the form of a straight or branched chain and contain 1 to 6 carbon atoms and the cycloalkyl radicals contain 3 to 10 carbon atoms,
their optical isomers and their pharmaceutically acceptable salts,
with the exception of the compound for which R₂ and R₃ represent phenyl radicals, R₁ represents a radical -N(R₅)-Y-R₆, Y is SO₂, R₅ represents a methyl radical and R₆ represents a phenyl radical.

6. Compounds of formula (I) according to Claim 5 for which Het is chosen from benzimidazole, benzoxazole, benzothiazole, benzothiophene, cinnoline, thiophene, quinazoline, quinoxaline, quinoline, pyrazole, pyrrole, pyridine, imidazole, indole, isoquinoline, pyrimidine, thiazole, thiadiazole, piperidine, piperazine, pyrrolidine, triazole, furan, tetrahydroisoquinoline, tetrahydroquinoline, these heterocycles being optionally substituted with one or more halogen atoms or alkyl, alkoxy, vinyl, alkoxycarbonyl, oxo, hydroxyl, OCF₃ or CF₃ radicals for which R₂ and R₃ represent phenyl radicals, R₁ represents a radical -N(R₅)-Y-R₆, Y is SO₂, R₅ represents a methyl radical and R₆ represents a phenyl radical.

7. Compounds of formula (I) according to Claim 5 in which
R₁ represents a radical -N(R₅)-Y-R₆,
Y is SO₂,
R₂ represents either a phenyl which is unsubstituted or substituted with one or more halogen atoms or alkyl, alkoxy, trifluoromethyl, trifluoromethoxy, cyano, -CONR₇R₈, hydroxyalkyl or -alk-NR₇R₈ radicals; or a heteroaromatic chosen from the pyridyl, pyrimidinyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈, -alk-NR₉R₁₀, alkylsulfanyl, alkylsulfinyl, alkylsulfonyl or hydroxyalkyl radical,
R₃ represents either a phenyl which is unsubstituted or substituted with one or more halogen atoms or alkyl, alkoxy, trifluoromethyl, trifluoromethoxy, cyano, -CONR₇R₈, hydroxyalkyl or -alk-NR₇R₈ radicals; or a heteroaromatic chosen from the pyridyl, pyrimidinyl, thiazolyl and thienyl rings, it being possible for these heteroaromatics to be unsubstituted or substituted with a halogen atom or an alkyl, alkoxy, hydroxyl, trifluoromethyl, trifluoromethoxy, -CONR₇R₈, -alk-NR₉R₁₀, alkylsulfanyl, alkylsulfinyl, alkylsulfonyl or hydroxyalkyl radical,
R₅ represents a hydrogen atom or an alkyl radical,
R₆ represents a naphthyl, phenylalkyl, Het or phenyl radical optionally substituted with one or more halogen atoms or alkyl, alkoxy, cyano, -CO-alk, COOalk, -CONR₁₂R₁₃, -alk-NR₁₄R₁₅, -NR₁₄R₁₅, hydroxyl, hydroxyalkyl, Het, OCF₃, CF₃, -NH-CO-alk, -SO₂NH₂ or -NH-COOalk radicals, or alternatively, on 2 adjacent carbon atoms, with dioxymethylene,
R₇ and R₈, which are identical or different, represent a hydrogen atom or an alkyl radical or alternatively R₇ and R₈ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₉ and R₁₀, which are identical or different, represent a hydrogen atom or an alkyl, cycloalkyl, alkylcycloalkyl or hydroxyalkyl radical or alternatively R₉ and R₁₀ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, oxo or -CO-NH₂ radicals,
R₁₂ and R₁₃, which are identical or different, represent a hydrogen atom or an alkyl radical or alternatively R₁₂ and R₁₃ together form with the nitrogen atom to which they are attached a 3- to 10-membered saturated mono- or bicyclic heterocycle, optionally containing another heteroatom chosen from oxygen, sulfur and nitrogen and being optionally substituted with one or more alkyl radicals,
R₁₄ and R₁₅, which are identical or different, represent a hydrogen atom or an alkyl, cycloalkyl, alkylcycloalkyl or hydroxyalkyl radical or alternatively R₁₄ and R₁₅ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, oxo, hydroxyalkyl or -CO-NH₂ radicals,
Het represents a 3- to 10-membered unsaturated or saturated mono- or bicyclic heterocycle containing one or more heteroatoms chosen from oxygen, sulfur and nitrogen optionally substituted with one or more halogen atoms or alkyl, alkoxy, vinyl, alkoxycarbonyl, oxo or hydroxyl radicals, the nitrogen-containing heterocycles being optionally in their N-oxidized form and, preferably, Het represents a heterocycle chosen from the following heterocycles: benzimidazole, benzoxazole, benzothiazole, benzothiophene, thiophene, quinazoline, quinoxaline, quinoline, pyrrole, pyridine, imidazole, indole, isoquinoline, pyrimidine, thiazole, thiadiazole, furan, tetrahydroisoquinoline and tetrahydroquinoline, these heterocycles being optionally substituted with one or more halogen atoms or alkyl, alkoxy, vinyl, oxo, hydroxyl, OCF₃ or CF₃ radicals,
their optical isomers and their pharmaceutically acceptable salts,
with the exception of the compound for which R₂ and R₃ represent phenyl radicals, R₁ represents a radical -N(R₅)-Y-R₆, Y is SO₂, R₅ represents a methyl radical and R₆ represents a phenyl radical.

8. Compounds of formula (I) according to Claim 5 in which
R₁ represents a radical -N(R₅)-Y-R₆,
Y is SO₂,
R₂ represents either a phenyl which is unsubstituted or substituted with one or more halogen atoms or alkyl, alkoxy, trifluoromethyl, trifluoromethoxy or hydroxyalkyl radicals; or a heteroaromatic chosen from pyridyl and pyrimidyl rings, it being possible for these heteroaromatics to be unsubstituted or substituted with a halogen atom or an alkyl, alkoxy, hydroxyl, trifluoromethyl or trifluoromethoxy radical, R₃ represents either a phenyl which is unsubstituted or substituted with one or more halogen atoms or alkyl, alkoxy, trifluoromethyl, trifluoromethoxy or hydroxyalkyl radicals; or a heteroaromatic chosen from pyridyl and pyrimidyl rings, it being possible for these heteroaromatics to be unsubstituted or substituted with a halogen atom or an alkyl, alkoxy, hydroxyl, trifluoromethyl or trifluoromethoxy radical, R₅ represents a hydrogen atom or an alkyl radical,
R₆ represents a naphthyl, phenylalkyl, Het or phenyl radical optionally substituted with one or more halogen atoms or alkyl, alkoxy, -NR₁₄R₁₅, hydroxyl, hydroxyalkyl, OCF₃, CF₃ or -SO₂NH₂ radicals, or alternatively, on 2 adjacent carbon atoms, with dioxymethylene,
R₁₄ and R₁₅, which are identical or different, represent a hydrogen atom or an alkyl, cycloalkyl, alkylcycloalkyl or hydroxyalkyl radical or alternatively R₁₄ and R₁₅ together form with the nitrogen atom to which they are attached a 3- to 10-membered saturated or unsaturated mono- or bicyclic heterocycle, optionally containing another heteroatom chosen from oxygen, sulfur and nitrogen and being optionally substituted with one or more alkyl, oxo, hydroxyalkyl or -CO-NH₂ radicals,
Het represents a 3- to 10-membered unsaturated or saturated mono- or bicyclic heterocycle containing one or more heteroatoms chosen from oxygen, sulfur and nitrogen optionally substituted with one or more halogen atoms or alkyl, alkoxy, vinyl, alkoxycarbonyl, oxo or hydroxyl radicals, the nitrogen-containing heterocycles being optionally in their N-oxidized form and, preferably, Het represents a heterocycle chosen from the following heterocycles: benzimidazole, benzoxazole, benzothiazole, benzothiophene, thiophene, quinoline, pyrrole, pyridine, pyrimidine, thiazole, thiadiazole, furan, tetrahydroisoquinoline and tetrahydroquinoline, these heterocycles being optionally substituted with one or more halogen atoms or alkyl, alkoxy, vinyl, oxo, hydroxyl, OCF₃ or CF₃ radicals,
their optical isomers and their pharmaceutically acceptable salts,
with the exception of the compound for which R₂ and R₃ represent phenyl radicals, R₁ represents a radical -N(R₅)-Y-R₆, Y is SO₂, R₅ represents a methyl radical and R₆ represents a phenyl radical.

9. Compound of formula (I) according to one of Claims 1 to 8, **characterized in that** it is chosen from:
• N-{1-[bis(4-chlorophenyl)methyl]azetidin-3-yl}-thien-2-yl-sulfonamide;
• N-{1-[bis(4-chlorophenyl)methyl]azetidin-3-yl}-4-methoxyphenylsulfonamide;
• N-[4-(N-{1-[bis(4-chlorophenyl)methyl]azetidin-3-yl}sulfamoyl)phenyl]acetamide;
• N-{1-[bis(4-chlorophenyl)methyl]azetidin-3-yl}-4-methylphenylsulfonamide;
• N-{1-[bis(4-chlorophenyl)methyl]azetidin-3-yl}-3,4-dimethoxyphenylsulfonamide;
• N-{1-[bis(4-chlorophenyl)methyl]azetidin-3-yl}-3-fluorophenylsulfonamide;
• N-{1-[bis(4-chlorophenyl)methyl]azetidin-3-yl}-3,4-dichlorophenylsulfonamide;
• N-{1-[bis(4-chlorophenyl)methyl]azetidin-3-yl}-3-cyanophenylsulfonamide;
• N-{1-[bis(4-chlorophenyl)methyl]azetidin-3-yl}-2,5-dimethoxyphenylsulfonamide;
• N-{1-[bis(4-chlorophenyl)methyl]azetidin-3-yl}-3-trifluoromethylphenylsulfonamide;
• N-{1-[bis(4-chlorophenyl)methyl]azetidin-3-yl}-naphth-2-ylsulfonamide;
• N-{1-[bis(4-chlorophenyl)methyl]azetidin-3-yl}-naphth-1-ylsulfonamide;
• N-{1-[bis(4-chlorophenyl)methyl]azetidin-3-yl}-3,4-difluorophenylsulfonamide;
• N-{1-[bis(4-chlorophenyl)methyl]azetidin-3-yl}-1-methyl-1-H-imidazol-4-ylsulfonamide;
• N-[4-(N-{1-[bis(4-chlorophenyl)methyl]azetidin-3-yl}sulfamoyl)-2-chlorophenyl]acetamide;
• N-{1-[bis(4-chlorophenyl)methyl]azetidin-3-yl}pyrid-3-ylsulfonamide;
• N-{1-[bis(4-chlorophenyl)methyl]azetidin-3-yl}-4-fluorophenylsulfonamide;
• N-{1-[bis(4-chlorophenyl)methyl]azetidin-3-yl}quinol-8-ylsulfonamide;
• N-{1-[bis(4-chlorophenyl)methyl]azetidin-3-yl}phenylsulfonamide;
• N-{1-[bis(4-chlorophenyl)methyl]azetidin-3-yl}-(phenylmethyl)sulfonamide;
• N-{1-[bis(4-chlorophenyl)methyl]azetidin-3-yl}-3,5-difluorophenylsulfonamide;
• N-{1-[bis(4-chlorophenyl)methyl]azetidin-3-yl}pyrid-2-ylsulfonamide;
• N-{1-[bis(4-chlorophenyl)methyl]azetidin-3-yl}-(3-fluoro-5-pyrrolidin-1-ylphenyl)sulfonamide;
• N-{1-[bis(4-chlorophenyl)methyl]azetidin-3-yl}-N-methyl-4-fluorophenylsulfonamide;
• N-{1-[bis(4-chlorophenyl)methyl]azetidin-3-yl}-N-methylquinol-8-ylsulfonamide;
• N-{1-[bis(4-chlorophenyl)methyl]azetidin-3-yl}-N-methylphenylsulfonamide;
• N-{1-[bis(4-chlorophenyl)methyl]azetidin-3-yl}-N-methyl(phenylmethyl)sulfonamide;
• N-{1-[bis(4-chlorophenyl)methyl]azetidin-3-yl}-3-sulfamoylphenylsulfonamide;
• 2-benzenesulfonyl-N-{1-[bis(4-chlorophenyl)-methyl]azetidin-3-yl}acetamide;
• N-{1-[bis(4-chlorophenyl)methyl]azetidin-3-yl}-2-(toluene-4-sulfonyl)acetamide;
• N-{1-[bis(4-chlorophenyl)methyl]azetidin-3-yl}-2-(toluene-4-sulfonyl)acetamide;
• N-{1-[bis(4-chlorophenyl)methyl]azetidin-3-yl}-3-(2-phenylethylenesulfonyl)propionamide;
• N-{1-[bis(4-chlorophenyl)methyl]azetidin-3-yl}-4-methanesulfonylbenzamide;
• N-{1-[bis(4-chlorophenyl)methyl]azetidin-3-yl}-4-methanesulfonylbenzamide;
• (5-methylsulfonylthiophene-2-carboxy){1-[bis(4-chlorophenyl)methyl]azetidin-3-yl}amide;
• (5-methylsulfonyl-3-methyl-4-vinylthiophene-2-carboxy){1-[bis(4-chlorophenyl)methyl]azetidin-3-yl}amide;
• N-{1-[bis(4-chlorophenyl)methyl]azetidin-3-yl}-3-methylsulfonylmethylbenzamide;
• (RS)-N-{1-[(4-chlorophenyl)pyridin-3-ylmethyl]-azetidin-3-yl}-3,5-difluorobenzenesulfonamide;
• (RS)-N-{1-[(4-chlorophenyl)pyrimidin-5-ylmethyl]-azetidin-3-yl}-3,5-difluorobenzenesulfonamide.

10. Process for preparing the compounds of formula (I) according to Claim 5 for which R₁ represents a radical -NHCOR₄, **characterized in that** an acid R₄COOH for which R₄ has the same meanings as in Claim 5 is reacted with a derivative of formula: 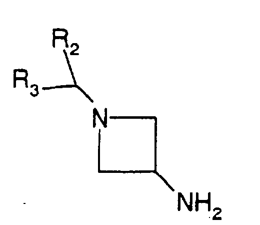 in which R₂ and R₃ have the same meanings as in Claim 5, the product isolated and optionally converted to a pharmaceutically acceptable salt.

11. Process for preparing the compounds of formula (I) according to Claim 5 for which R₁ represents a radical -N(R₅)-Y-R₆, **characterized in that** a derivative of formula: 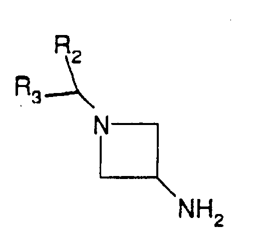 in which R₂ and R₃ have the same meanings as in Claim 5 is reacted with a derivative Hal-Y-R₆ for which Y and R₆ have the same meanings as in Claim 5 and Hal represents a halogen atom, optionally followed by a derivative Hal-alk, Hal represents a halogen atom and alk represents an alkyl radical (1-6C in the form of a straight or branched chain) to give the compounds for which R₅ is an alkyl radical, the product isolated and optionally converted to a pharmaceutically acceptable salt.

12. Process for preparing the compounds of formula (I) according to Claim 5, **characterized in that** a derivative R₂-CHBr-R₃ for which R₂ and R₃ have the same meanings as in Claim 5 is reacted with a derivative of formula: 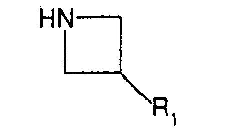 in which R₁ has the same meanings as in Claim 5, the product isolated and optionally converted to a pharmaceutically acceptable salt.

13. Process for preparing the compounds of formula (I) according to Claim 5 for which R₁ represents a radical -N(R₅)-Y-R₆ in which R₆ is a phenyl radical substituted with a hydroxyl radical, **characterized in that** a corresponding compound of formula (I) for which R₁ represents a radical -N(R₅)-Y-R₆ in which R₆ is a phenyl radical substituted with an alkoxy radical is hydrolyzed, the product isolated and optionally converted to a pharmaceutically acceptable salt.

14. Process for preparing the compounds of formula (I) according to Claim 5 for which R₁ represents a radical -N(R₅)-Y-R₆ in which R₆ is a phenyl radical substituted with a hydroxy(1C)alkyl radical, **characterized in that** diisobutylaluminum hydride is reacted with a corresponding compound of formula (I) for which R₁ represents a radical -N(R₅)-Y-R₆ in which R₆ is a phenyl radical substituted with an alkoxycarbonyl radical, the product isolated and optionally converted to a pharmaceutically acceptable salt.

15. Process for preparing the compounds of formula (I) according to Claim 5 for which R₁ represents a radical -N(R₅)-Y-R₆ in which R₆ is a phenyl radical substituted with a 1-pyrrolidinyl radical, **characterized in that** pyrrolidine is reacted with a corresponding compound of formula (I) for which R₁ represents a radical -N(R₅)-Y-R₆ in which R₆ is a phenyl radical substituted with a fluorine atom, the product isolated and optionally converted to a pharmaceutically acceptable salt.

16. Medicament, **characterized in that** it contains, as active ingredient, at least one compound of formula (I) according to one of Claims 1 to 9.

## Patentansprüche

1. Pharmazeutische Zusammensetzung, enthaltend als Wirkstoff eine Verbindung der Formel: 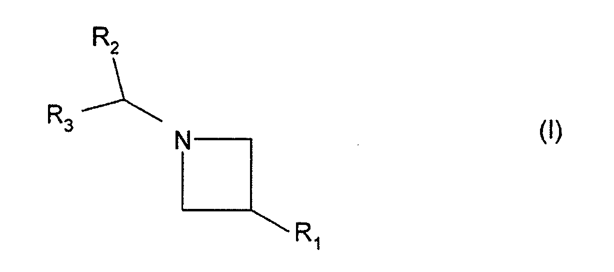 worin
R₁ für einen -NHCOR₄- oder -N(R₅)-Y-R₆-Rest steht,
Y für CO oder SO₂ steht,
R₂ and R₃ gleich oder verschieden sind und entweder für einen unter Phenyl, Naphthyl und Indenyl ausgewählten Aromaten, wobei diese Aromaten unsubstituiert oder durch einen oder mehrere Halogen-, Alkyl-, Alkoxy-, Formyl-, Hydroxyl-, Trifluormethyl-, Trifluormethoxy-, -CO-alk-, Cyano-, -COOH-, -COOalk-, -CONR₇R₈-, -CO-NH-NR₉R₁₀-, Alkylsulfanyl-, Alkylsulfinyl-, Alkylsulfonyl-, Alkylsulfanylalkyl-, Alkylsulfinylalkyl-, Alkylsulfonylalkyl-, Hydroxyalkyl- oder -alk-NR₇R₈-Reste substituiert sind; oder einen unter Benzofuryl-, Benzothiazolyl-, Benzothienyl-, Benzoxazolyl-, Chromanyl-, 2,3-Dihydrobenzofuryl-, 2,3-Dihydrobenzothienyl-, Pyrimidinyl-, Furyl-, Imidazolyl-, Isochromanyl-, Isochinolyl-, Pyrrolyl-, Pyridyl-, Chinolyl-, 1,2,3,4-Tetrahydroisochinolyl-, Thiazolyl- und Thienylringen ausgewählten Heteroaromaten, wobei diese Heteroaromaten unsubstituiert oder durch einen Halogen-, Alkyl-, Alkoxy-, Hydroxyl-, Trifluormethyl-, Trifluormethoxy-, Cyano-, -COOH-, -COOalk-, -CO-NH-NR₉R₁₀-, -CONR₇R₈-, -alk-NR₉R₁₀-, Alkylsulfanyl-, Alkylsulfinyl-, Alkylsulfonyl-, Alkylsulfanylalkyl-, Alkylsulfinylalkyl-, Alkylsulfonylalkyl- oder Hydroxyalkylrest substituiert sein können, stehen;
R₄ für einen -alk-SO₂-R₁₁-, -alk-SO₂-CH=CH-R₁₁-, durch -SO₂-R₁₁ substituierten Het- oder durch -SO₂-R₁₁ oder -alk-SO₂-R₁₁ substituierten Phenylrest steht,
R₅ für ein Wasserstoffatom oder einen Alkylrest steht,
R₆ für einen Phenylalkyl-, Het- oder Ar-Rest steht, R₇ und R₈ gleich oder verschieden sind und für ein Wasserstoffatom oder einen Alkylrest stehen oder auch gemeinsam mit dem Stickstoffatom, an das sie gebunden sind, einen 3- bis 10-gliedrigen gesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkylreste substituiert ist,
R₉ und R₁₀ gleich oder verschieden sind und für ein Wasserstoffatom oder einen Alkyl-, -COOalk-, Cycloalkyl-, Alkylcycloalkyl-, -alk-O-alk- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, -COalk-, -COOalk-, -CO-NHalk-, -CS-NHalk-, Oxo-, Hydroxyalkyl-, -alk-O-alk- oder -CO-NH₂-Reste substituiert ist,
R₁₁ für einen Alkyl-, Ar- oder Het-Rest steht,
Ar für einen Phenyl-, Naphthyl- oder Indenylrest steht, wobei diese Reste gegebenenfalls durch einen oder mehrere Halogen-, Alkyl-, Alkoxy-, Cyano-, -CO-alk-, -COOH-, -COOalk-, -CONR₁₂R₁₃-, -CO-NH-NR₁₄R₁₅-, Alkylsulfanyl-, Alkylsulfinyl-, Alkylsulfonyl-, -alk-NR₁₄R₁₅-, -NR₁₄R₁₅-, Alkylthioalkyl-, Formyl-, Hydroxyl-, Hydroxyalkyl-, Het-, -O-alk-NH-Cycloalkyl-, OCF₃-, CF₃-, -NH-CO-alk-, -SO₂NH₂-, -NH-COCH₃-, -NH-COOalk- oder Het-Reste oder auch an 2 benachbarten Kohlenstoffatomen durch einen Dioxymethylenrest substituiert sind,
Het für einen 3- bis 10-gliedrigen ungesättigten oder gesättigten mono- oder bicyclischen Heterocyclus steht, der ein oder mehrere unter Sauerstoff, Schwefel und Stickstoff ausgewählte Heteroatome enthält und gegebenenfalls durch einen oder mehrere Alkyl-, Alkoxy-, Vinyl-, Halogen-, Alkoxycarbonyl-, Oxo-, Hydroxyl-, OCF₃- oder CF₃-Reste substituiert ist, wobei die stickstoffhaltigen Heterocyclen gegebenenfalls in ihrer N-Oxid-Form vorliegen,
R₁₂ und R₁₃ gleich oder verschieden sind und für ein Wasserstoffatom oder einen Alkylrest stehen oder auch gemeinsam mit dem Stickstoffatom, an das sie gebunden sind, einen 3- bis 10-gliedrigen gesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkylreste substituiert ist,
R₁₄ und R₁₅ gleich oder verschieden sind und für ein Wasserstoffatom oder einen Alkyl-, -COOalk-, Cycloalkyl-, Alkylcycloalkyl-, -alk-O-alk- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, -COalk-, -COOalk-, -CO-NHalk-, -CS-NHalk-, Oxo-, Hydroxyalkyl-, -alk-O-alk- oder -CO-NH₂-Reste substituiert ist,
alk für einen Alkyl- oder Alkylenrest steht,
die Alkyl- und Alkylenreste und -teile und Alkoxyreste und -teile gerad- oder verzweigtkettig sind und 1 bis 6 Kohlenstoffatome enthalten und die Cycloalkylreste 3 bis 10 Kohlenstoffatome enthalten,
ein optisches Isomer einer derartigen Verbindung oder eines ihrer pharmazeutisch unbedenklichen Salze.

2. Zusammensetzung nach Anspruch 1, für die in der Verbindung der Formel (I) Het unter Benzimidazol, Benzoxazol, Benzothiazol, Benzothiophen, Cinnolin, Thiophen, Chinazolin, Chinoxalin, Chinolin, Pyrazol, Pyrrol, Pyridin, Imidazol, Indol, Isochinolin, Pyrimidin, Thiazol, Thiadiazol, Piperidin, Piperazin, Pyrrolidin, Triazol, Furan, Tetrahydroisochinolin und Tetrahydrochinolin ausgewählt ist, wobei diese Heterocyclen gegebenenfalls durch einen oder mehrere Alkyl-, Alkoxy-, Vinyl-, Halogen-, Alkoxycarbonyl-, Oxo-, Hydroxyl-, OCF₃- oder CF₃-Reste substituiert sind.

3. Pharmazeutische Zusammensetzung, enthaltend als Wirkstoff mindestens eine Verbindung der Formel (I) nach Anspruch 1, worin
R₁ für einen -N(R₅)-Y-R₆-Rest steht,
Y für SO₂ steht,
R₂ entweder für Phenyl, das unsubstituiert oder durch einen oder mehrere Halogen-, Alkyl-, Alkoxy-, Trifluormethyl-, Trifluormethoxy-, Cyano-, -CONR₇R₈-, Hydroxyalkyl- oder -alk-NR₇R₈-Reste substituiert ist; oder einen unter Pyridyl-, Pyrimidinyl-, Thiazolyl- und Thienylringen ausgewählten Heteroaromaten, wobei diese Heteroaromaten unsubstituiert oder durch einen Halogen-, Alkyl-, Alkoxy-, Hydroxyl-, Trifluormethyl-, Trifluormethoxy-, -CONR₇R₈-, -alk-NR₉R₁₀-, Alkylsulfanyl-, Alkylsulfinyl-, Alkylsulfonyl- oder Hydroxyalkylrest substituiert sein können, steht,
R₃ entweder für Phenyl, das unsubstituiert oder durch einen oder mehrere Halogen-, Alkyl-, Alkoxy-, Trifluormethyl-, Trifluormethoxy-, Cyano-, -CONR₇R₈-, Hydroxyalkyl- oder -alk-NR₇R₈-Reste substituiert ist; oder einen unter Pyridyl-, Pyrimidinyl-, Thiazolyl- und Thienylringen ausgewählten Heteroaromaten, wobei diese Heteroaromaten unsubstituiert oder durch einen Halogen-, Alkyl-, Alkoxy-, Hydroxyl-, Trifluormethyl-, Trifluormethoxy-, -CONR₇R₈-, -alk-NR₉R₁₀-, Alkylsulfanyl-, Alkylsulfinyl-, Alkylsulfonyl- oder Hydroxyalkylrest substituiert sein können, steht,
R₅ für ein Wasserstoffatom oder einen Alkylrest steht,
R₆ für einen Naphthyl-, Phenylalkyl-, Het- oder Phenylrest steht, der gegebenenfalls durch einen oder mehrere Halogen-, Alkyl-, Alkoxy-, Cyano-, -CO-alk-, -COOalk-, -CONR₁₂R₁₃-, -alk-NR₁₄R₁₅-, -NR₁₄R₁₅-, Hydroxyl-, Hydroxyalkyl-, Het-, OCF₃-, CF₃-, -NH-CO-alk-, -SO₂NH₂- oder -NH-COOalk-Reste oder auch an 2 benachbarten Kohlenstoffatomen durch einen Dioxymethylenrest substituiert ist,
R₇ und R₈ gleich oder verschieden sind und für ein Wasserstoffatom oder einen Alkylrest stehen oder auch gemeinsam mit dem Stickstoffatom, an das sie gebunden sind, einen 3- bis 10-gliedrigen gesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkylreste substituiert ist,
R₉ und R₁₀ gleich oder verschieden sind und für ein Wasserstoffatom oder einen Alkyl-, Cycloalkyl-, Alkylcycloalkyl- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, Oxo- oder -CO-NH₂-Reste substituiert ist,
R₁₂ und R₁₃ gleich oder verschieden sind und für ein Wasserstoffatom oder einen Alkylrest stehen oder auch gemeinsam mit dem Stickstoffatom, an das sie gebunden sind, einen 3- bis 10-gliedrigen gesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkylreste substituiert ist,
R₁₄ und R₁₅ gleich oder verschieden sind und für ein Wasserstoffatom oder einen Alkyl-, Cycloalkyl-, Alkylcycloalkyl- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, Oxo-, Hydroxyalkyl- oder -CO-NH₂-Reste substituiert ist,
Het für einen 3- bis 10-gliedrigen ungesättigten oder gesättigten mono- oder bicyclischen Heterocyclus steht, der ein oder mehrere unter Sauerstoff, Schwefel und Stickstoff ausgewählte Heteroatome enthält und gegebenenfalls durch einen oder mehrere Alkyl-, Alkoxy-, Vinyl-, Halogen-, Alkoxycarbonyl-, Oxo- oder Hydroxyreste substituiert ist, wobei die stickstoffhaltigen Heterocyclen gegebenenfalls in ihrer N-Oxid-Form vorliegen, und vorzugsweise für einen unter den folgenden Heterocyclen ausgewählten Heterocyclus steht: Benzimidazol, Benzoxazol, Benzothiazol, Benzothiophen, Thiophen, Chinazolin, Chinoxalin, Chinolin, Pyrrol, Pyridin, Imidazol, Indol, Isochinolin, Pyrimidin, Thiazol, Thiadiazol, Furan, Tetrahydroisochinolin und Tetrahydrochinolin, wobei diese Heterocyclen gegebenenfalls durch einen oder mehrere Alkyl-, Alkoxy-, Vinyl-, Halogen-, Oxo-, Hydroxyl-, OCF₃- oder CF₃-Reste substituiert sind,
ein optisches Isomer einer derartigen Verbindung oder eines ihrer pharmazeutisch unbedenklichen Salze.

4. Pharmazeutische Zusammensetzung, enthaltend als Wirkstoff mindestens eine Verbindung der Formel (I) nach Anspruch 1, worin
R₁ für einen -N(R₅)-Y-R₆-Rest steht,
Y für SO₂ steht,
R₂ entweder für Phenyl, das unsubstituiert oder durch einen oder mehrere Halogen-, Alkyl-, Alkoxy-, Trifluormethyl-, Trifluormethoxy- oder Hydroxyalkylreste substituiert ist; oder einen unter Pyridyl- und Pyrimidinylringen ausgewählten Heteroaromaten, wobei diese Heteroaromaten unsubstituiert oder durch einen Halogen-, Alkyl-, Alkoxy-, Hydroxyl-, Trifluormethyl- oder Trifluormethoxyrest substituiert sein können, steht,
R₃ entweder für Phenyl, das unsubstituiert oder durch einen oder mehrere Halogen-, Alkyl-, Alkoxy-, Trifluormethyl-, Trifluormethoxy- oder Hydroxyalkylreste substituiert ist; oder einen unter Pyridyl- und Pyrimidinylringen ausgewählten Heteroaromaten, wobei diese Heteroaromaten unsubstituiert oder durch einen Halogen-, Alkyl-, Alkoxy-, Hydroxyl-, Trifluormethyl- oder Trifluormethoxyrest substituiert sein können, steht,
R₅ für ein Wasserstoffatom oder einen Alkylrest steht,
R₆ für einen Naphthyl-, Phenylalkyl-, Het- oder Phenylrest steht, der gegebenenfalls durch einen oder mehrere Halogen-, Alkyl-, Alkoxy-, -NR₁₄R₁₅-, Hydroxyl-, Hydroxyalkyl-, OCF₃-, CF₃- oder -SO₂NH₂-Reste oder auch an 2 benachbarten Kohlenstoffatomen durch einen Dioxymethylenrest substituiert ist,
R₁₄ und R₁₅ gleich oder verschieden sind und für ein Wasserstoffatom oder einen Alkyl-, Cycloalkyl-, Alkylcycloalkyl- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, Oxo-, Hydroxyalkyl- oder -CO-NH₂-Reste substituiert ist,
Het für einen 3- bis 10-gliedrigen ungesättigten oder gesättigten mono- oder bicyclischen Heterocyclus steht, der ein oder mehrere unter Sauerstoff, Schwefel und Stickstoff ausgewählte Heteroatome enthält und gegebenenfalls durch einen oder mehrere Alkyl-, Alkoxy-, Vinyl-, Halogen-, Alkoxycarbonyl-, Oxo- oder Hydroxyreste substituiert ist, wobei die stickstoffhaltigen Heterocyclen gegebenenfalls in ihrer N-Oxid-Form vorliegen, und vorzugsweise für einen unter den folgenden Heterocyclen ausgewählten Heterocyclus steht: Benzimidazol, Benzoxazol, Benzothiazol, Benzothiophen, Thiophen, Chinolin, Pyrrol, Pyridin, Pyrimidin, Thiazol, Thiadiazol, Furan, Tetrahydroisochinolin und Tetrahydrochinolin, wobei diese Heterocyclen gegebenenfalls durch einen oder mehrere Alkyl-, Alkoxy-, Vinyl-, Halogen-, Oxo-, Hydroxyl-, OCF₃- oder CF₃-Reste substituiert sind,
ein optisches Isomer einer derartigen Verbindung oder eines ihrer pharmazeutisch unbedenklichen Salze.

5. Verbindungen der Formel: 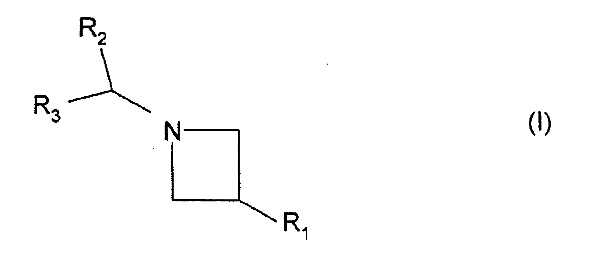 worin
R₁ für einen -NHCOR₄- oder -N(R₅)-Y-R₆-Rest steht,
Y für CO oder SO₂ steht,
R₂ and R₃ gleich oder verschieden sind und entweder für einen unter Phenyl, Naphthyl und Indenyl ausgewählten Aromaten, wobei diese Aromaten unsubstituiert oder durch einen oder mehrere Halogen-, Alkyl-, Alkoxy-, Formyl-, Hydroxyl-, Trifluormethyl-, Trifluormethoxy-, -CO-alk-, Cyano-, -COOH-, -COOalk-, -CONR₇R₈-, -CO-NH-NR₉R₁₀-, Alkylsulfanyl-, Alkylsulfinyl-, Alkylsulfonyl-, Alkylsulfanylalkyl-, Alkylsulfinylalkyl-, Alkylsulfonylalkyl-, Hydroxyalkyl- oder -alk-NR₇R₈-Reste substituiert sind; oder einen unter Benzofuryl-, Benzothiazolyl-, Benzothienyl-, Benzoxazolyl-, Chromanyl-, 2,3-Dihydrobenzofuryl-, 2,3-Dihydrobenzothienyl-, Pyrimidinyl-, Furyl-, Imidazolyl-, Isochromanyl-, Isochinolyl-, Pyrrolyl-, Pyridyl-, Chinolyl-, 1,2,3,4-Tetrahydroisochinolyl-, Thiazolyl- und Thienylringen ausgewählten Heteroaromaten, wobei diese Heteroaromaten unsubstituiert oder durch einen Halogen-, Alkyl-, Alkoxy-, Hydroxyl-, Trifluormethyl-, Trifluormethoxy-, Cyano-, -COOH-, -COOalk-, -CO-NH-NR₉R₁₀-, -CO-NR₇R₈-, -alk-NR₉R₁₀-, Alkylsulfanyl-, Alkylsulfinyl-, Alkylsulfonyl-, Alkylsulfanylalkyl-, Alkylsulfinylalkyl-, Alkylsulfonylalkyl- oder Hydroxyalkylrest substituiert sein können, stehen;
R₄ für einen -alk-SO₂-R₁₁-, -alk-SO₂-CH=CH-R₁₁-, durch -SO₂-R₁₁ substituierten Het- oder durch -SO₂-R₁₁ oder -alk-SO₂-R₁₁ substituierten Phenylrest steht,
R₅ für ein Wasserstoffatom oder einen Alkylrest steht,
R₆ für einen Phenylalkyl-, Het- oder Ar-Rest steht, R₇ und R₈ gleich oder verschieden sind und für ein Wasserstoffatom oder einen Alkylrest stehen oder auch gemeinsam mit dem Stickstoffatom, an das sie gebunden sind, einen 3- bis 10-gliedrigen gesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkylreste substituiert ist,
R₉ und R₁₀ gleich oder verschieden sind und für ein Wasserstoffatom oder einen Alkyl-, -COOalk-, Cycloalkyl-, Alkylcycloalkyl-, -alk-O-alk- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, -COalk-, -COOalk-, -CO-NHalk-, -CS-NHalk-, Oxo-, Hydroxyalkyl-, -alk-O-alk- oder -CO-NH₂-Reste substituiert ist,
R₁₁ für einen Alkyl-, Ar- oder Het-Rest steht,
Ar für einen Phenyl-, Naphthyl- oder Indenylrest steht, wobei diese Reste gegebenenfalls durch einen oder mehrere Halogen-, Alkyl-, Alkoxy-, Cyano-, -CO-alk-, -COOH-, -COOalk-, -CONR₁₂R₁₃-, -CO-NH-NR₁₄R₁₅-, Alkylsulfanyl-, Alkylsulfinyl-, Alkylsulfonyl-, -alk-NR₁₄R₁₅-, -NR₁₄R₁₅-, Alkylthioalkyl-, Formyl-, Hydroxyl-, Hydroxyalkyl-, Het-, -O-alk-NH-Cycloalkyl-, OCF₃-, CF₃-, -NH-CO-alk-, -SO₂NH₂-, -NH-COCH₃-, -NH-COOalk- oder Het-Reste oder auch an 2 benachbarten Kohlenstoffatomen durch einen Dioxymethylenrest substituiert sind,
Het für einen 3- bis 10-gliedrigen ungesättigten oder gesättigten mono- oder bicyclischen Heterocyclus steht, der ein oder mehrere unter Sauerstoff, Schwefel und Stickstoff ausgewählte Heteroatome enthält und gegebenenfalls durch einen oder mehrere Alkyl-, Alkoxy-, Vinyl-, Halogen-, Alkoxycarbonyl-, Oxo-, Hydroxyl-, OCF₃- oder CF₃-Reste substituiert ist, wobei die stickstoffhaltigen Heterocyclen gegebenenfalls in ihrer N-Oxid-Form vorliegen,
R₁₂ und R₁₃ gleich oder verschieden sind und für ein Wasserstoffatom oder einen Alkylrest stehen oder auch gemeinsam mit dem Stickstoffatom, an das sie gebunden sind, einen 3- bis 10-gliedrigen gesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkylreste substituiert ist,
R₁₄ und R₁₅ gleich oder verschieden sind und für ein Wasserstoffatom oder einen Alkyl-, -COOalk-, Cycloalkyl-, Alkylcycloalkyl-, -alk-O-alk- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, -COalk-, -COOalk-, -CO-NHalk-, -CS-NHalk-, Oxo-, Hydroxyalkyl-, -alk-O-alk- oder -CO-NH₂-Reste substituiert ist,
alk für einen Alkyl- oder Alkylenrest steht,
die Alkyl- und Alkylenreste und -teile und Alkoxyreste und -teile gerad- oder verzweigtkettig sind und 1 bis 6 Kohlenstoffatome enthalten und die Cycloalkylreste 3 bis 10 Kohlenstoffatome enthalten,
ihre optischen Isomere und ihre pharmazeutisch unbedenklichen Salze,
mit Ausnahme der Verbindung, für die R₂ und R₃ für Phenylreste stehen, R₁ für einen -N(R₅)-Y-R₆-Rest steht, Y für SO₂ steht, R₅ für einen Methylrest steht und R₆ für einen Phenylrest steht.

6. Verbindungen der Formel (I) nach Anspruch 5, für die Het unter Benzimidazol, Benzoxazol, Benzothiazol, Benzothiophen, Cinnolin, Thiophen, Chinazolin, Chinoxalin, Chinolin, Pyrazol, Pyrrol, Pyridin, Imidazol, Indol, Isochinolin, Pyrimidin, Thiazol, Thiadiazol, Piperidin, Piperazin, Pyrrolidin, Triazol, Furan, Tetrahydroisochinolin und Tetrahydrochinolin ausgewählt ist, wobei diese Heterocyclen gegebenenfalls durch einen oder mehrere Alkyl-, Alkoxy-, Vinyl-, Halogen-, Alkoxycarbonyl-, Oxo-, Hydroxyl-, OCF₃- oder CF₃-Reste substituiert sind, für die R₂ und R₃ für Phenylreste stehen, R₁ für einen -N(R₅)-Y-R₆-Rest steht, Y für SO₂ steht, R₅ für einen Methylrest steht und R₆ für einen Phenylrest steht.

7. Verbindungen der Formel (I) nach Anspruch 5, worin
R₁ für einen -N(R₅)-Y-R₆-Rest steht,
Y für SO₂ steht,
R₂ entweder für Phenyl, das unsubstituiert oder durch einen oder mehrere Halogen-, Alkyl-, Alkoxy-, Trifluormethyl-, Trifluormethoxy-, Cyano-, -CONR₇R₈-, Hydroxyalkyl- oder -alk-NR₇R₈-Reste substituiert ist; oder einen unter Pyridyl-, Pyrimidinyl-, Thiazolyl- und Thienylringen ausgewählten Heteroaromaten, wobei diese Heteroaromaten unsubstituiert oder durch einen Halogen-, Alkyl-, Alkoxy-, Hydroxyl-, Trifluormethyl-, Trifluormethoxy-, -CONR₇R₈-, -alk-NR₉R₁₀-, Alkylsulfanyl-, Alkylsulfinyl-, Alkylsulfonyl- oder Hydroxyalkylrest substituiert sein können, steht,
R₃ entweder für Phenyl, das unsubstituiert oder durch einen oder mehrere Halogen-, Alkyl-, Alkoxy-, Trifluormethyl-, Trifluormethoxy-, Cyano-, -CONR₇R₈-, Hydroxyalkyl- oder -alk-NR₇R₈-Reste substituiert ist; oder einen unter Pyridyl-, Pyrimidinyl-, Thiazolyl- und Thienylringen ausgewählten Heteroaromaten, wobei diese Heteroaromaten unsubstituiert oder durch einen Halogen-, Alkyl-, Alkoxy-, Hydroxyl-, Trifluormethyl-, Trifluormethoxy-, -CONR₇R₈-, -alk-NR₉R₁₀-, Alkylsulfanyl-, Alkylsulfinyl-, Alkylsulfonyl- oder Hydroxyalkylrest substituiert sein können, steht,
R₅ für ein Wasserstoffatom oder einen Alkylrest steht,
R₆ für einen Naphthyl-, Phenylalkyl-, Het- oder Phenylrest steht, der gegebenenfalls durch einen oder mehrere Halogen-, Alkyl-, Alkoxy-, Cyano-, -CO-alk-, -COOalk-, -CONR₁₂R₁₃-, -alk-NR₁₄R₁₅-, -NR₁₄R₁₅-, Hydroxyl-, Hydroxyalkyl-, Het-, OCF₃-, CF₃-, -NH-CO-alk-, -SO₂NH₂- oder -NH-COOalk-Reste oder auch an 2 benachbarten Kohlenstoffatomen durch einen Dioxymethylenrest substituiert ist,
R₇ und R₈ gleich oder verschieden sind und für ein Wasserstoffatom oder einen Alkylrest stehen oder auch gemeinsam mit dem Stickstoffatom, an das sie gebunden sind, einen 3- bis 10-gliedrigen gesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkylreste substituiert ist,
R₉ und R₁₀ gleich oder verschieden sind und für ein Wasserstoffatom oder einen Alkyl-, Cycloalkyl-, Alkylcycloalkyl- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, Oxo- oder -CO-NH₂-Reste substituiert ist,
R₁₂ und R₁₃ gleich oder verschieden sind und für ein Wasserstoffatom oder einen Alkylrest stehen oder auch gemeinsam mit dem Stickstoffatom, an das sie gebunden sind, einen 3- bis 10-gliedrigen gesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkylreste substituiert ist,
R₁₄ und R₁₅ gleich oder verschieden sind und für ein Wasserstoffatom oder einen Alkyl-, Cycloalkyl-, Alkylcycloalkyl- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, Oxo-, Hydroxyalkyl- oder -CO-NH₂-Reste substituiert ist,
Het für einen 3- bis 10-gliedrigen ungesättigten oder gesättigten mono- oder bicyclischen Heterocyclus steht, der ein oder mehrere unter Sauerstoff, Schwefel und Stickstoff ausgewählte Heteroatome enthält und gegebenenfalls durch einen oder mehrere Alkyl-, Alkoxy-, Vinyl-, Halogen-, Alkoxycarbonyl-, Oxo- oder Hydroxyreste substituiert ist, wobei die stickstoffhaltigen Heterocyclen gegebenenfalls in ihrer N-Oxid-Form vorliegen, und vorzugsweise für einen unter den folgenden Heterocyclen ausgewählten Heterocyclus steht: Benzimidazol, Benzoxazol, Benzothiazol, Benzothiophen, Thiophen, Chinazolin, Chinoxalin, Chinolin, Pyrrol, Pyridin, Imidazol, Indol, Isochinolin, Pyrimidin, Thiazol, Thiadiazol, Furan, Tetrahydroisochinolin und Tetrahydrochinolin, wobei diese Heterocyclen gegebenenfalls durch einen oder mehrere Alkyl-, Alkoxy-, Vinyl-, Halogen-, Oxo-, Hydroxyl-, OCF₃- oder CF₃-Reste substituiert sind,
ihre optischen Isomere und ihre pharmazeutisch unbedenklichen Salze,
mit Ausnahme der Verbindung, für die R₂ und R₃ für Phenylreste stehen, R₁ für einen -N(R₅)-Y-R₆-Rest steht, Y für SO₂ steht, R₅ für einen Methylrest steht und R₆ für einen Phenylrest steht.

8. Verbindungen der Formel (I) nach Anspruch 5, worin
R₁ für einen -N(R₅)-Y-R₆-Rest steht,
Y für SO₂ steht,
R₂ entweder für Phenyl, das unsubstituiert oder durch einen oder mehrere Halogen-, Alkyl-, Alkoxy-, Trifluormethyl-, Trifluormethoxy- oder Hydroxyalkylreste substituiert ist; oder einen unter Pyridyl- und Pyrimidinylringen ausgewählten Heteroaromaten, wobei diese Heteroaromaten unsubstituiert oder durch einen Halogen-, Alkyl-, Alkoxy-, Hydroxyl-, Trifluormethyl- oder Trifluormethoxyrest substituiert sein können, steht,
R₃ entweder für Phenyl, das unsubstituiert oder durch einen oder mehrere Halogen-, Alkyl-, Alkoxy-, Trifluormethyl-, Trifluormethoxy- oder Hydroxyalkylreste substituiert ist; oder einen unter Pyridyl- und Pyrimidinylringen ausgewählten Heteroaromaten, wobei diese Heteroaromaten unsubstituiert oder durch einen Halogen-, Alkyl-, Alkoxy-, Hydroxyl-, Trifluormethyl- oder Trifluormethoxyrest substituiert sein können, steht,
R₅ für ein Wasserstoffatom oder einen Alkylrest steht,
R₆ für einen Naphthyl-, Phenylalkyl-, Het- oder Phenylrest steht, der gegebenenfalls durch einen oder mehrere Halogen-, Alkyl-, Alkoxy-, -NR₁₄R₁₅-, Hydroxyl-, Hydroxyalkyl-, OCF₃-, CF₃- oder -SO₂NH₂-Reste oder auch an 2 benachbarten Kohlenstoffatomen durch einen Dioxymethylenrest substituiert ist,
R₁₄ und R₁₅ gleich oder verschieden sind und für ein Wasserstoffatom oder einen Alkyl-, Cycloalkyl-, Alkylcycloalkyl- oder Hydroxyalkylrest stehen oder auch gemeinsam mit dem Stickstoffatom, an das sie gebunden sind, einen 3- bis 10-gliedrigen gesättigten oder ungesättigten mono- oder bicyclischen Heterocyclus bilden, der gegebenenfalls ein weiteres unter Sauerstoff, Stickstoff und Schwefel ausgewähltes Heteroatom enthält und gegebenenfalls durch einen oder mehrere Alkyl-, Oxo-, Hydroxyalkyl- oder -CO-NH₂-Reste substituiert ist,
Het für einen 3- bis 10-gliedrigen ungesättigten oder gesättigten mono- oder bicyclischen Heterocyclus steht, der ein oder mehrere unter Sauerstoff, Schwefel und Stickstoff ausgewählte Heteroatome enthält und gegebenenfalls durch einen oder mehrere Alkyl-, Alkoxy-, Vinyl-, Halogen-, Alkoxycarbonyl-, Oxo- oder Hydroxyreste substituiert ist, wobei die stickstoffhaltigen Heterocyclen gegebenenfalls in ihrer N-Oxid-Form vorliegen, und vorzugsweise für einen unter den folgenden Heterocyclen ausgewählten Heterocyclus steht: Benzimidazol, Benzoxazol, Benzothiazol, Benzothiophen, Thiophen, Chinolin, Pyrrol, Pyridin, Pyrimidin, Thiazol, Thiadiazol, Furan, Tetrahydroisochinolin und Tetrahydrochinolin, wobei diese Heterocyclen gegebenenfalls durch einen oder mehrere Alkyl-, Alkoxy-, Vinyl-, Halogen-, Oxo-, Hydroxyl-, OCF₃- oder CF₃-Reste substituiert sind,
ihre optischen Isomere und ihre pharmazeutisch unbedenklichen Salze,
mit Ausnahme der Verbindung, für die R₂ und R₃ für Phenylreste stehen, R₁ für einen -N(R₅)-Y-R₆-Rest steht, Y für SO₂ steht, R₅ für einen Methylrest steht und R₆ für einen Phenylrest steht.

9. Verbindung der Formel (I) nach einem der Ansprüche 1 bis 8, **dadurch gekennzeichnet, daß** sie ausgewählt ist unter:
• N-{1-[Bis(4-chlorphenyl)methyl]azetidin-3-yl}-thien-2-yl-sulfonamid;
• N-{1-[Bis(4-chlorphenyl)methyl]azetidin-3-yl}-4-methoxyphenylsulfonamid;
• N-[4-(N-{1-[Bis(4-chlorphenyl)methyl]azetidin-3-yl}sulfamoyl)phenyl]acetamid;
• N-{1-[Bis(4-chlorphenyl)methyl]azetidin-3-yl}-4-methylphenylsulfonamid;
• N-{1-[Bis(4-chlorphenyl)methyl]azetidin-3-yl}-3,4-dimethoxyphenylsulfonamid;
• N-{1-[Bis(4-chlorphenyl)methyl]azetidin-3-yl}-3-fluorphenylsulfonamid;
• N-{1-[Bis(4-chlorphenyl)methyl]azetidin-3-yl}-3,4-dichlorphenylsulfonamid;
• N-{1-[Bis(4-chlorphenyl)methyl]azetidin-3-yl}-3-cyanophenylsulfonamid;
• N-{1-[Bis(4-chlorphenyl)methyl]azetidin-3-yl}-2,5-dimethoxyphenylsulfonamid;
• N-{1-[Bis(4-chlorphenyl)methyl]azetidin-3-yl}-3-trifluormethylphenylsulfonamid;
• N-{1-[Bis(4-chlorphenyl)methyl]azetidin-3-yl}-naphth-2-yl-sulfonamid;
• N-{1-[Bis(4-chlorphenyl)methyl]azetidin-3-yl}-naphth-1-yl-sulfonamid;
• N-{1-[Bis(4-chlorphenyl)methyl]azetidin-3-yl}-3,4-difluorphenylsulfonamid;
• N-{1-[Bis(4-chlorphenyl)methyl]azetidin-3-yl}-1-methyl-1-H-imidazol-4-yl-sulfonamid;
• N-[4-(N-{1-[Bis(4-chlorphenyl)methyl]azetidin-3-yl}sulfamoyl)-2-chlorphenyl]acetamid;
• N-{1-[Bis(4-chlorphenyl)methyl]azetidin-3-yl}pyrid-3-yl-sulfonamid;
• N-{1-[Bis(4-chlorphenyl)methyl]azetidin-3-yl}-4-fluorphenylsulfonamid;
• N-{1-[Bis(4-chlorphenyl)methyl]azetidin-3-yl}chinol-8-ylsulfonamid;
• N-{1-[Bis(4-chlorphenyl)methyl]azetidin-3-yl}-phenylsulfonamid;
• N-{1-[Bis(4-chlorphenyl)methyl]azetidin-3-yl}-(phenylmethyl)sulfonamid;
• N-{1-[Bis(4-chlorphenyl)methyl]azetidin-3-yl}-3,5-difluorphenylsulfonamid;
• N-{1-[Bis(4-chlorphenyl)methyl]azetidin-3-yl}-pyrid-2-ylsulfonamid;
• N-{1-[Bis(4-chlorphenyl)methyl]azetidin-3-yl}-(3-fluor-5-pyrrolidin-1-ylphenyl)sulfonamid;
• N-{1-[Bis(4-chlorphenyl)methyl]azetidin-3-yl}-N-methyl-4-fluorphenylsulfonamid;
• N-{1-[Bis(4-chlorphenyl)methyl]azetidin-3-yl}-N-methylchinol-8-ylsulfonamid;
• N-{1-[Bis(4-chlorphenyl)methyl]azetidin-3-yl}-N-methylphenylsulfonamid;
• N-{1-[Bis(4-chlorphenyl)methyl]azetidin-3-yl}-N-methyl(phenylmethyl)sulfonamid;
• N-{1-[Bis(4-chlorphenyl)methyl]azetidin-3-yl}-3-sulfamoylphenylsulfonamid;
• 2-Benzolsulfonyl-N-{1-[bis(4-chlorphenyl)-methyl]azetidin-3-yl}acetamid;
• N-{1-[Bis(4-chlorphenyl)methyl]azetidin-3-yl}-2-(toluol-4-sulfonyl)acetamid;
• N-{1-[Bis(4-chlorphenyl)methyl]azetidin-3-yl}-2-(toluol-4-sulfonyl)acetamid;
• N-{1-[Bis(4-chlorphenyl)methyl]azetidin-3-yl}-3-(2-phenylethylensulfonyl)propionamid;
• N-{1-[Bis(4-chlorphenyl)methyl]azetidin-3-yl}-4-methansulfonylbenzamid;
• N-{1-[Bis(4-chlorphenyl)methyl]azetidin-3-yl}-4-methansulfonylbenzamid;
• (5-Methylsulfonylthiophen-2-carboxy)-{1-[bis(4-chlorphenyl)methyl]azetidin-3-yl}amid;
• (5-Methylsulfonyl-3-methyl-4-vinylthiophen-2-carboxy)-{1-[bis(4-chlorphenyl)methyl]azetidin-3-yl}amid;
• N-{1-[Bis(4-chlorphenyl)methyl]azetidin-3-yl}-3-methylsulfonylmethylbenzamid;
• (RS)-N-{1-[(4-Chlorphenyl)pyridin-3-ylmethyl]-azetidin-3-yl}-3,5-difluorbenzolsulfonamid;
• (RS)-N-{1-[(4-Chlorphenyl)pyrimidin-5-ylmethyl]-azetidin-3-yl}-3,5-difluorbenzolsulfonamid.

10. Verfahren zur Herstellung von Verbindungen der Formel (I) nach Anspruch 5, für die R₁ für einen -NHCOR₄-Rest steht, **dadurch gekennzeichnet, daß** man eine Säure R₄COOH, für die R₄ die gleichen Bedeutungen besitzt wie in Anspruch 5, mit einem Derivat der Formel: 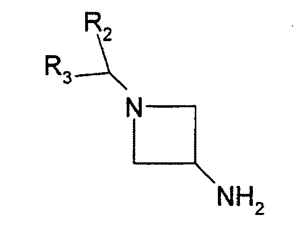 worin R₂ und R₃ die gleichen Bedeutungen besitzen wie in Anspruch 5, umsetzt und das Produkt isoliert und gegebenenfalls in ein pharmazeutisch unbedenkliches Salz umwandelt.

11. Verfahren zur Herstellung von Verbindungen der Formel (I) nach Anspruch 5, für die R₁ für einen -N(R₅)-Y-R₆-Rest steht, **dadurch gekennzeichnet, daß** man ein Derivat der Formel: 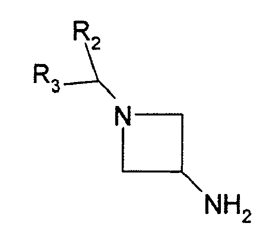 worin R₂ und R₃ die gleichen Bedeutungen besitzen wie in Anspruch 5, mit einem Derivat Hal-Y-R₆, für das Y und R₆ die gleichen Bedeutungen besitzen wie in Anspruch 5 und Hal für ein Halogenatom steht, und gegebenenfalls zum Erhalt von Verbindungen, für die R₅ für Alkyl steht, danach mit einem Derivat Hal-alk, für das Hal für ein Halogenatom steht und alk für einen gerad- oder verzweigtkettigen C₁₋₆-Alkylrest steht, umsetzt und das Produkt isoliert und gegebenenfalls in ein pharmazeutisch unbedenkliches Salz umwandelt.

12. Verfahren zur Herstellung von Verbindungen der Formel (I) nach Anspruch 5, **dadurch gekennzeichnet, daß** man ein Derivat R₂-CHBr-R₃, für das R₂ und R₃ die gleichen Bedeutungen besitzen wie in Anspruch 5, mit einem Derivat der Formel: 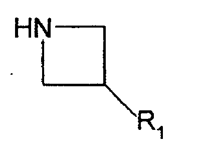 worin R₁ die gleichen Bedeutungen besitzt wie in Anspruch 5, umsetzt und das Produkt isoliert und gegebenenfalls in ein pharmazeutisch unbedenkliches Salz umwandelt.

13. Verfahren zur Herstellung von Verbindungen der Formel (I) nach Anspruch 5, für die R₁ für einen -N(R₅)-Y-R₆-Rest steht, worin R₆ für einen durch Hydroxyl substituierten Phenylrest steht, **dadurch gekennzeichnet, daß** man eine entsprechende Verbindung der Formel (I), für die R₁ für einen -N(R₅)-Y-R₆-Rest steht, worin R₆ für einen durch Alkoxy substituierten Phenylrest steht, hydrolysiert und das Produkt isoliert und gegebenenfalls in ein pharmazeutisch unbedenkliches Salz umwandelt.

14. Verfahren zur Herstellung von Verbindungen der Formel (I) nach Anspruch 5, für die R₁ für einen -N(R₅)-Y-R₆-Rest steht, worin R₆ für einen durch C₁-Hydroxyalkyl substituierten Phenylrest steht, **dadurch gekennzeichnet, daß** man Diisobutylaluminiumhydrid mit einer entsprechenden Verbindung der Formel (I), für die R₁ für einen -N(R₅)-Y-R₆-Rest steht, worin R₆ für einen durch Alkoxycarbonyl substituierten Phenylrest steht, umsetzt und das Produkt isoliert und gegebenenfalls in ein pharmazeutisch unbedenkliches Salz umwandelt.

15. Verfahren zur Herstellung von Verbindungen der Formel (I) nach Anspruch 5, für die R₁ für einen -N(R₅)-Y-R₆-Rest steht, worin R₆ für einen durch 1-Pyrrolidinyl substituierten Phenylrest steht, **dadurch gekennzeichnet, daß** man Pyrrolidin mit einer entsprechenden Verbindung der Formel (I), für die R₁ für einen -N(R₅)-Y-R₆-Rest steht, worin R₆ für einen durch Fluor substituierten Phenylrest steht, umsetzt und das Produkt isoliert und gegebenenfalls in ein pharmazeutisch unbedenkliches Salz umwandelt.

16. Arzneimittel, **dadurch gekennzeichnet, daß** es als Wirkstoff mindestens eine Verbindung der Formel (I) nach einem der Ansprüche 1 bis 9 enthält.
